(19) Europäisches Patentamt
European Patent Office
Office européen des brevets

(11) EP 2 295 438 B2

(12) # NEW EUROPEAN PATENT SPECIFICATION
After opposition procedure

(45) Date of publication and mention of the opposition decision:
**26.03.2025 Bulletin 2025/13**

(45) Mention of the grant of the patent:
**18.03.2015 Bulletin 2015/12**

(21) Application number: **09754828.3**

(22) Date of filing: **29.05.2009**

(51) International Patent Classification (IPC):
***C07D 487/08*** $^{(2006.01)}$ ***C07D 295/08*** $^{(2006.01)}$

(52) Cooperative Patent Classification (CPC):
**C07D 295/08; C07D 487/08; C08G 18/1825; C08G 18/2063; C08G 18/7621;** C08G 2110/0083

(86) International application number:
**PCT/JP2009/059903**

(87) International publication number:
**WO 2009/145320 (03.12.2009 Gazette 2009/49)**

(54) **PROCESS FOR PRODUCING HYDROXYALKYLTRIETHYLENEDIAMINE COMPOUND, AND CATALYST COMPOSITION FOR THE PRODUCTION OF POLYURETHANE RESIN USING THE HYDROXYALKYLTRIETHYLENEDIAMINE COMPOUND**

VERFAHREN ZUR HERSTELLUNG EINER HYDROXYALKYLTRIETHYLENDIAMINVERBINDUNG UND KATALYSATORZUSAMMENSETZUNG FÜR DIE HERSTELLUNG VON POLYURETHANHARZ MIT DER HYDROXYALKYLTRIETHYLENDIAMINVERBINDUNG

PROCESSUS DESTINÉ À PRODUIRE UN COMPOSÉ HYDROXYALKYLTRIÉTHYLÈNEDIAMINE, ET HYDROXYALKYLTRIÉTHYLÈNEDIAMINE DESTINÉE À PRODUIRE DE LA RÉSINE DE POLYURÉTHANE À L AIDE DU COMPOSÉ HYDROXYALKYLTRIÉTHYLÈNEDIAMINE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority:
**30.05.2008 JP 2008142586**
**09.07.2008 JP 2008178990**
**16.07.2008 JP 2008185165**
**07.08.2008 JP 2008204535**
**29.10.2008 JP 2008278254**
**31.10.2008 JP 2008281558**
**20.11.2008 JP 2008296910**
**21.11.2008 JP 2008297912**

(43) Date of publication of application:
**16.03.2011 Bulletin 2011/11**

(73) Proprietor: **Tosoh Corporation**
**Yamaguchi 746-8501 (JP)**

(72) Inventors:
- **TOKUMOTO, Katsumi**
**Shunan-shi**
**Yamaguchi 746-8501 (JP)**
- **SUZUKI, Takao**
**Shunan-shi**
**Yamaguchi 746-8501 (JP)**
- **KISO, Hiroyuki**
**Shunan-shi**
**Yamaguchi 746-8501 (JP)**
- **TAKAHASHI, Yoshihiro**
**Shunan-shi**
**Yamaguchi 746-8501 (JP)**
- **TAMANO, Yutaka**
**Shunan-shi**
**Yamaguchi 746-8501 (JP)**

(74) Representative: **Vigand, Philippe et al**
**Novagraaf International SA**
**Chemin de l'Echo 3**
**1213 Onex - Genève (CH)**

(56) References cited:
**WO-A2-2009/030649** **AT-B- 227 268**
**AT-B- 227 268** **CA-A1- 2 609 308**
**CN-A- 1 478 780** **FR-A1- 2 756 563**
**JP-A- 2001 139 546** **JP-A- 2003 082 051**
**JP-A- 2003 082 052** **JP-A- 2003 082 052**
**JP-A- 2003 313 164** **JP-A- 2006 526 670**
**JP-A- 2008 056 849** **JP-A- 2010 037 488**
**JP-A- 2010 105 944** **JP-A- 2010 106 192**
**JP-A- 2010 120 887** **JP-A- H03 176 463**
**JP-A- H06 239 952** **JP-A- S63 259 565**
**US-A- 2 400 022** **US-A- 3 167 555**
**US-A- 3 297 701** **US-A- 3 520 835**
**US-A- 4 026 840** **US-A- 4 590 223**

| | |
|---|---|
| US-A- 5 071 809 | US-A- 5 143 944 |
| US-A- 5 710 191 | US-A- 6 057 321 |
| US-A- 6 147 185 | US-A1- 2003 083 393 |

- Dissertation Jens Bietz, December 2008
- Yang, Peng Fei. "Effects of Solvent Polarity on the Urethane Reaction of 1,2-Propanediol." Advanced Materials Research, vol. 450-451, Trans Tech Publications, Ltd., Jan. 2012, pp. 38-41
- DATABASE CA [online] CHEMICAL ABSTRACTS SERVICE, COLUMBUS, OHIO, US; INOUE, SHINICHI ET AL: "Low-toxic catalyst compositions for polyurethanes with high polymerization activity and manufacture of polyurethanes", XP002674935, retrieved from STN Database accession no. 2003:216973
- LABADIE, J.W. ET AL.: "Selective catalytic syntheses of mixed alkyl amines", JOURNAL OF MOLECULAR CATALYSIS, vol. 42, no. 3, 1987, pages 367 - 378, XP008146439
- SMITH M.B. ET AL., MARCH'S ADVANCED ORGANIC CHEMISTRY: REACTIONS, MECHANISMS, AND STRUCTURE, 2001, pages 423, 118

## Description

TECHNICAL FIELD

The present invention relates to a catalyst composition for the production of a polyurethane resin, which comprises a hydroxyalkyltriethylenediamine or hydroxytriethylenediamine, and a process for producing a polyurethane resin, which uses the catalyst composition.

BACKGROUND ART

Hydroxyalkyltriethylenediamines or hydroxytriethylenediamine is a compound useful for e.g. intermediates for medicines or agricultural chemicals, catalysts for organic syntheses, chemical adsorbents or fungicidal agents.

Whereas, hydroxyalkylpiperazines are compounds useful for e.g. intermediates for medicines or agricultural chemicals, catalysts for organic syntheses, chemical adsorbents or fungicidal agents.

Further, a catalyst composition containing a hydroxyalkyltriethylenediamine or hydroxytriethylenediamine is very useful as a catalyst for the production of a polyurethane resin, which has substantially no volatile amine catalyst or hazardous metal catalyst at the time of producing a polyurethane resin.

As a process for producing a hydroxyalkyltriethylenediamine or hydroxytriethylenediamine represented by the following formula (2a):

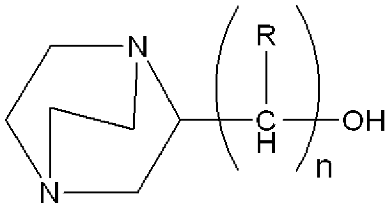

(2a)

(wherein R is a hydrogen atom or a linear or branched $C_{1-4}$ alkyl group, and n is an integer of from 0 to 6), a process has, for example, been known wherein piperazine and ethyl 2,3-dibromopropanoate are reacted to prepare 1,4-diazabicyclo [2.2.2]octane-2-carboxylic acid ethyl ester, and then, the obtained ester is reduced to obtain 1,4-diazabicyclo[2.2.2] octane-2-methanol (i.e. hydroxymethyltriethylenediamine) (e.g. Patent Document 1).

However, such a process is industrially disadvantageous, since it requires multistage reaction steps.

Further, in the above process, a by-product salt is formed in a large amount in the first step, whereby purification becomes cumbersome, and a low substrate concentration is required, whereby the productivity tends to be poor. Further, in the second step, lithium aluminum hydride having a high risk of catching fire is employed as a reducing agent, such being undesirable from the viewpoint of safety. Further, a strong reducing agent such as lithium aluminum hydride is required to be carefully post-treated after completion of the reaction, such being industrially disadvantageous. Further, an expensive reaction substrate is used, such being practically disadvantageous.

On the other hand, as a process for producing a hydroxyalkylpiperazine, a process has been known wherein ethylenediamine and dihydroxyacetone are reacted, followed by hydrogen reduction in the presence of a catalyst to obtain 2-hydroxymethylpiperazine (2-piperazine methanol) (e.g. Patent Document 2).

However, this process cannot be regarded as an industrial process, since it requires a high pressure reaction, and the reaction yield is as low as at most 40%.

Further, a process has been known wherein dibenzylethylenediamine and diethylbromomalonic acid are reacted in acetonitrile, followed by hydrogen reduction in the presence of a noble metal catalyst, and the ester is reduced by means of lithium aluminum hydride to obtain 4-benzyl-2-hydroxymethylpiperazine (e.g. Non-Patent Document 1).

However, this process requires a multistage reaction, and when the reaction is carried out in three stages, the total yield is as low as 44%, and such a process cannot be regarded as an industrial process.

Whereas a polyurethane resin is produced by reacting a polyol with a polyisocyanate in the presence of a catalyst and, if required, a blowing agent, a surfactant, a flame retardant, a crosslinking agent, etc. For the production of polyurethane resins, it is known to use many metal-type compounds or tertiary amine compounds, as catalysts. Such catalysts are also industrially commonly used alone or in combination.

In the production of a polyurethane foam using water and/or a low boiling point organic compound as a blowing agent, among the above catalysts, a tertiary amine compound is especially widely used, since it is excellent in the productivity and moldability. Such a tertiary amine compound may, for example, be conventional triethylenediamine, N,N,N',N'-tetramethyl-1,6-hexanediamine, bis(2-dimethylaminoethyl) ether, N,N,N',N",N"-pentamethyldiethylenetriamine, N-methylmorpholine, N-ethylmorpholine or N,N-dimethylethanolamine (e.g. Non-Patent Document 1).

Further, as the metal-type compound, an organic metal compound such as an organic tin compound, may, for example, be frequently used. However, as the productivity or moldability tends to deteriorate, in most cases, it is used in combination with a tertiary amine catalyst, and it is rare that such an organic metal compound is used alone.

The above-mentioned tertiary amine compound is gradually discharged as a volatile amine from a polyurethane product, and accordingly, it brings about, for example, an odor problem due to the volatile amine in the case of e.g. interior material for automobiles, discoloration of PVC (vinyl chloride resin) of an instrument panel for automobiles or a fogging phenomenon of a window glass by migration of a volatile component from a polyurethane product (foam). Further, a tertiary amine compound as a catalyst usually has a strong offensive odor and thus very much deteriorates the working environment during the production of a polyurethane resin.

As a method to solve such problems, it has been proposed to use, instead of the above-described volatile tertiary amine compound, an amine catalyst (hereinafter sometimes referred to as a reactive catalyst) having a hydroxy group or primary or secondary amino group reactive with a polyisocyanate, in its molecule, or a bifunctional crosslinking agent having a tertiary amino group in its molecule (e.g. Patent Documents 3 to 7).

The method of using such a reactive catalyst is said to avoid the above problems, since the catalyst is fixed in the polyurethane resin backbone as reacted with the polyisocyanate. This method is certainly effective to reduce the odor of the final resin product, but such a reactive catalyst is inferior in the activity for gelling reaction (the reaction of a polyol with an isocyanate), and it has a problem that the curing property tends to be low.

Whereas the method of using the above-mentioned crosslinking agent is effective to reduce the odor of the final resin product and to improve the working environment during the production of a polyurethane resin, but the physical property such as the hardness of the polyurethane resin tends to be inadequate.

Further, a method has been proposed wherein an amine compound having a hydroxy group, a primary amino group and a secondary amino group in its molecule, is used as a catalyst for the production of a rigid polyurethane foam (e.g. Patent Documents 8 and 9), but such a method is intended to improve the flowability and thermal conductivity of a foam, and no study has been made to overcome the odor problem.

On the other hand, a metal-type compound will not bring about an odor problem or a problem of deteriorating other materials, like the above-described tertiary amine compound, but when such a metal-type compound is used alone, the productivity, physical properties and moldability tend to deteriorate as mentioned above, and further, an environmental problem due to a heavy metal remaining in the product has been pointed out.

PRIOR ART DOCUMENTS

PATENT DOCUMENTS

Patent Document 1: JP-A-2001-504855
Patent Document 2: Austrian Patent No. 227268
Patent Document 3: JP-A-46-4846
Patent Document 4: JP-B-61-31727
Patent Document 5: Japanese Patent No. 2,971,979
Patent Document:6: JP-A-63-265909
Patent Document 7: JP-A-2008-45113
Patent Document 8: JP-A-2003-82051
Patent Document 9: JP-A-2003-105051

NON-PATENT DOCUMENT

Non-Patent Document 1: Journal of Medicinal Chemistry (1993), 36(15), 2075-2083
Non-Patent Document 2: Keiji Iwata "Polyurethane Resin Handbook" (1987 first edition), Nikkan Kogyo Shimbun, Ltd., p. 118

JP-A-2003 082052 discloses a low toxic catalyst which can improve high-speed hardening and film properties of a coat in the manufacturing method of polyurethane resin usin an aliphatic isocyanate, and more particularly a catalyst composition comprising a combination of a metal complex catalyst such as $M(acac)_n$ (wherein M is Fe, Mn, Cu, Zr, Th, Ti or Co; acac represents acetylacetonate; n is an integer of $\geq 1$ and $\leq 4$) and a tertiary amine selected from triethylenediamine and 2-methyl-triethylenediamine.

DISCLOSURE OF THE INVENTION

OBJECTS TO BE ACCOMPLISHED BY THE INVENTION

The present invention has been made in view of the above-described background art, and the object of the present invention is to provide a novel catalyst composition capable of obtaining a polyurethane product with good productivity and moldability without bringing about an odor problem or an environmental problem, and a process for producing a polyurethane resin, employing such a catalyst composition.

MEANS TO ACCOMPLISH THE OBJECTS

The present inventors have carried out an extensive study to accomplish the above objects and as a result, have accomplished the present invention.

That is, the present invention provides a catalyst composition for the production of a polyurethane resin, containing a hydroxyalkyltriethylenediamine, and a process for producing a polyurethane resin, which uses the catalyst composition.

[1] A catalyst composition for the production of a polyurethane resin, which comprises :

a hydroxyalkyltriethylenediamine or hydroxytriethylenediamine (A), wherein the hydroxyalkyltriethylenediamine or hydroxytriethylenediamine (A) is one or more selected from the group consisting of
a hydroxyalkyltriethylenediamine or hydroxytriethylenediamine represented by the following formula (2a):

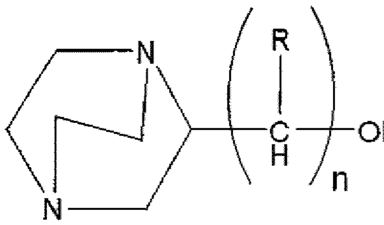

(2a)

where in the above formula (2a), R is a hydrogen atom or a linear or branched $C_{1-4}$ alkyl group, and n is an integer of from 0 to 6,
a hydroxyalkyltriethylenediamine represented by the following formula (2b):

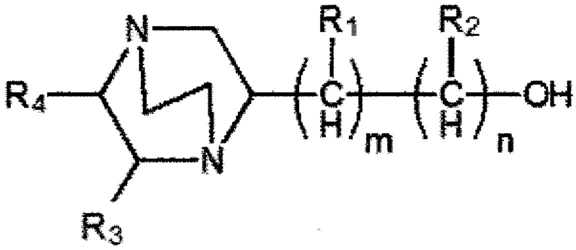

(2b)

where in the above formula (2b), each of $R_1$ to $R_4$ which are independent of each other, is a hydrogen atom or a linear or branched $C_{1-4}$ alkyl group, and each of m and n which are independent of each other, is an integer of from 0 to 2, provided m+n < 4, and/or
a hydroxyalkyltriethylenediamine represented by the following formula (2c):

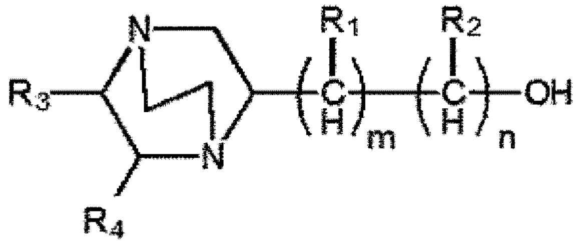

(2c)

where in the above formula (2c), each of $R_1$ to $R_4$ which are independent of each other, is a hydrogen atom or a linear or branched $C_{1-4}$ alkyl group, and each of m and n which are independent of each other, is an integer of from 0 to 2, provided m+n < 4, and
a hydroxymethyltriethylenediamine represented by the following formula (2d):

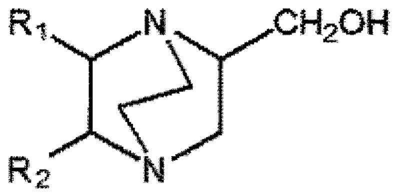

(2d)

where in the above formula (2d), each of $R_1$ and $R_2$ which are independent of each other, is a hydrogen atom or a $C_{1-4}$ alkyl group; and
an amine compound (B) having, in its molecule, one or more substituents selected from the group consisting of a hydroxy group, a primary amino group and a secondary amino group,
wherein the amine compound (B) having, in its molecule, one or more substituents selected from the group consisting of a hydroxy group, a primary amino group and a secondary amino group, is an amine compound represented by the following formula (11):

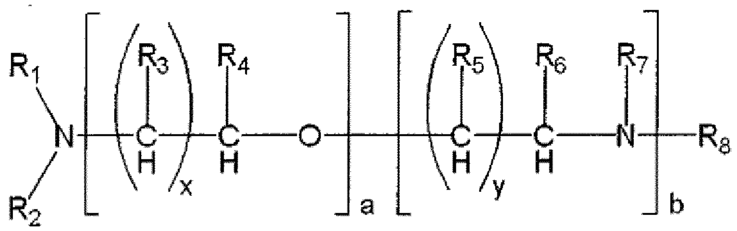

(11)

where in the above formula (11), each of $R_1$ to $R_8$ which are independent of each other, is a hydrogen atom, a hydroxyl group, a $C_{1-16}$ alkyl group, a $C_{6-16}$ aryl group, a $C_{1-10}$ hydroxyalkyl group, a $C_{1-10}$ aminoalkyl group, a $C_{1-10}$ monomethylaminoalkyl group or a $C_{1-10}$ dimethylaminoalkyl group, x is an integer of from 0 to 11, y is an integer of from 0 to 11, a is an integer of from 0 to 10 and b is an integer of from 0 to 10, and
wherein the amine compound represented by the above formula (11) is one or more amines selected from the group consisting of N,N-dimethylethylenediamine, N,N'-dimethylethylenediamine, N,N-dimethylpropylenediamine, N,N'-dimethylpropylenediamine, N,N-dimethylhexamethylenediamine, N,N'-dimethylhexamethylenediamine, trimethyldiethylenetriamine, trimethylethylenediamine, trimethylpropylenediamine, trimethylhexamethylenediamine, tetramethyldiethylenetriamine, N,N-dimethylaminoethanol, N,N-dimethylaminoisopropanol, bis(3-dimethylaminopropyl)amine, N-methylpiperazine, N,N-dimethylaminoethoxyethanol, N,N,N'-trimethylaminoethylethanolamine, N,N-dimethylaminoethyl-N'-methylaminoethyl-N"-methylaminoisopropanol, N,N-dimethylaminoethoxyethoxyethanol, N,N-dimethyl-N',N'-bis(2-hydroxypropyl)-1,3-propanediamine, N,N,N'-trimethyl-N'-(2-hydroxyethyl)bis(2-aminoethyl) ether, N,N-bis(3-dimethylaminopropyl)-N-isopropanolamine, N,N-dimethylaminohexanol and N,N,N'-trimethyl-N'-(2-hydroxyethyl)propylenediamine.

[2] The catalyst composition for the production of a polyurethane resin according to the above [1], wherein the hydroxyalkyltriethylenediamine or hydroxytriethylenediamine (A) is an amine compound represented by the following

formula (2e):

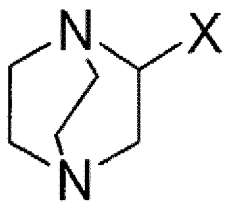

(2e)

where in the above formula (2e), X is a hydroxyl group, a hydroxymethyl group or a hydroxyethyl group.

[3] The catalyst composition for the production of a polyurethane resin according to any one of the above [1] to [2], wherein the mixed ratio of the hydroxyalkyltriethylenediamine or hydroxytriethylenediamine (A), to the amine compound (B) having, in its molecule, one or more substituents selected from the group consisting of a hydroxy group, a primary amino group and a secondary amino group, is amine compound (A)] / amine compound (B) = 1/99 to 99/1 weight ratio.

[4] A process for producing a polyurethane resin, which comprises reacting a polyol with a polyisocyanate in the presence of the catalyst composition as defined in any one of the above [1] to [3].

[5] The process for producing a polyurethane resin according to the above [4], wherein the catalyst composition as defined in any one of the above [1] to [3], is used in an amount within a range of from 0.01 to 30 parts by weight per 100 parts by weight of the polyol.

The present application also describes (I) a process for producing a hydroxyalkyltriethylene diamine or hydroxytriethylenediamine and (II) a process for producing a hydroxyalkylpiperazine and/or hydroxypiperazine not forming part of the present invention.

(I-1) Process for producing hydroxyalkyltriethylenediamine or hydroxytriethylenediamine not forming part of the present invention :

[10] A process for producing a hydroxyalkyltriethylenediamine or hydroxytriethylenediamine, which comprises subjecting a mono-substituted dihydroxyalkylpiperazine and/or a di-substituted hydroxyalkylpiperazine to an intramolecular dehydration condensation reaction in the presence of an acid catalyst.

[11] The process according to the above [10], wherein the acid catalyst comprises one or more compounds selected from the group consisting of a metal phosphate and an organic phosphorus compound.

[12] The process according to the above [10] or [11], wherein a mono-substituted dihydroxyalkylpiperazine represented by the following formula (1a):

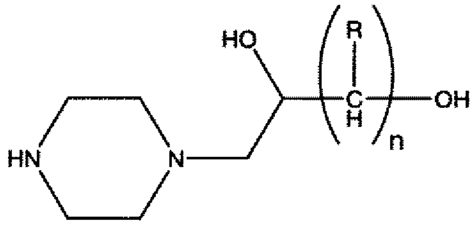

(1a)

[in the above formula (1a), R is a hydrogen atom or a linear or branched $C_{1-4}$ alkyl group, and n is an integer of from 0 to 6], or the following formula (1b):

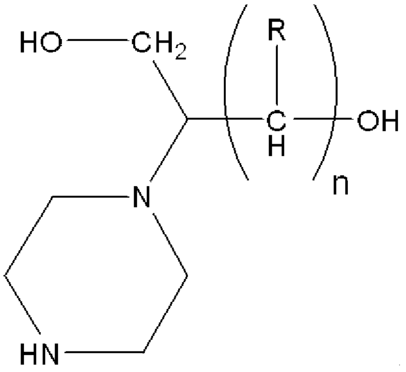

(1b)

[in the above formula (1b), R and n are the same as defined in the above formula (1a)], is subjected to an intramolecular dehydration condensation reaction in the presence of an acid catalyst, to obtain a hydroxyalkyltriethylenediamine or hydroxytriethylenediamine represented by the following formula (2a):

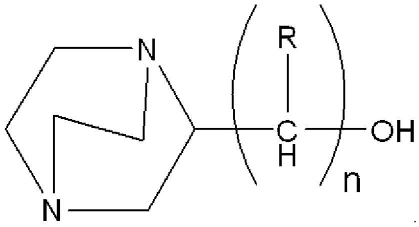

(2a)

[in the above formula (2a), R and n are the same as defined in the above formula (1a)].

[13] The process according to the above [12], wherein the mono-substituted dihydroxyalkylpiperazine represented by the above formula (1a) is a mono-substituted dihydroxyalkylpiperazine obtained by an addition reaction of piperazine with a compound represented by the following formula (4a):

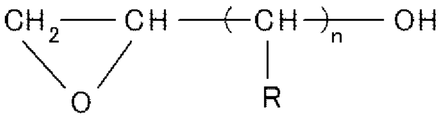

(4a)

[in the above formula (4a), R and n are the same as defined in the above formula (1a)].

[14] The process according to the above [12], wherein the mono-substituted dihydroxyalkylpiperazine represented by the above formula (1a) is a mono-substituted dihydroxyalkylpiperazine obtained by a dehydration condensation reaction of piperazine with a compound represented by the following formula (4b):

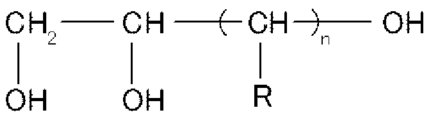

(4b)

[in the above formula (4b), R and n are the same as defined in the above formula (1a)] in the presence of an acid catalyst.

[15] The process according to the above [12], wherein the mono-substituted dihydroxyalkylpiperazine represented by the above formula (1a) is a mono-substituted dihydroxyalkylpiperazine obtained by a reaction of piperazine with a compound represented by the following formula (4c):

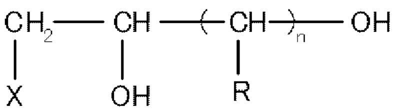

(4c)

[in the above formula (4c), R and n are the same as defined in the above formula (1a)].

[16] The process according to the above [12], wherein the mono-substituted dihydroxyalkylpiperazine represented by the above formula (1b) is a mono-substituted dihydroxyalkylpiperazine obtained by a reaction of piperazine with a compound represented by the following formula (5a):

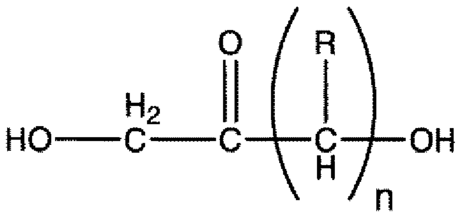

(5a)

[in the above formula (5a), R and n are the same as defined in the above formula (1a)].

[17] The process according to the above [12], wherein the mono-substituted dihydroxyalkylpiperazine represented by the above formula (1b) is a mono-substituted dihydroxyalkylpiperazine obtained by a reduction reaction of a dialkyl ester of piperazine which is obtained by a reaction of piperazine with a compound represented by the following formula (5b):

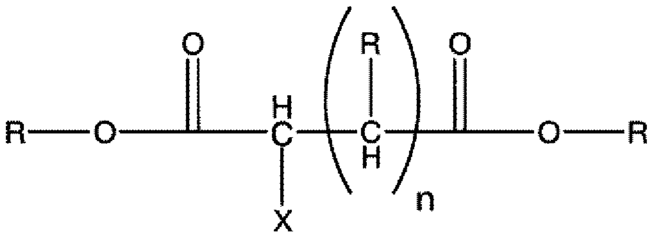

(5 b)

[in the above formula (5b), R and n are the same as defined in the above formula (1a)].

[18] The process according to the above [10] or [11], wherein a hydroxyalkylpiperazine or hydroxypiperazine represented by the following formula (6):

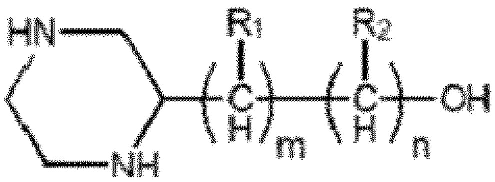

(6)

[in the above formula (6), each of $R_1$ and $R_2$ which are independent of each other, is a hydrogen atom or a linear or branched $C_{1-4}$ alkyl group, and each of m and n which are independent of each other, is an integer of from 0 to 2, provided m+n<4], is reacted with an alkylene oxide represented by the following formula (7):

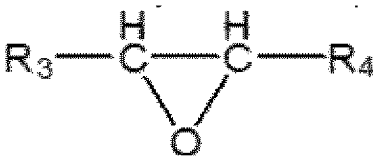

(7)

[in the above formula (7), each of $R_3$ and $R_4$ which are independent of each other, is a hydrogen atom or a linear or branched $C_{1-4}$ alkyl group] to obtain a di-substituted hydroxyalkylpiperazine represented by the following formula (1c):

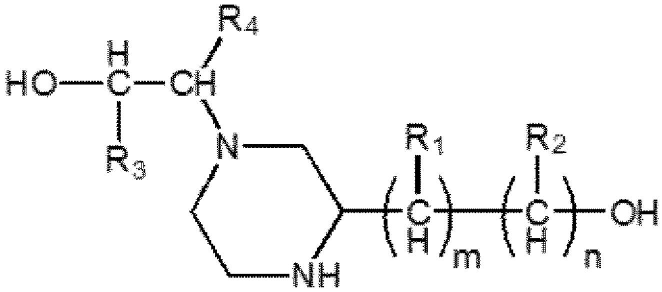

(1c)

[in the above formula (1c), $R_1$ to $R_4$ , m and n are the same as defined in the above formulae (6) and (7)] and/or a di-substituted hydroxyalkylpiperazine represented by the following formula (1d):

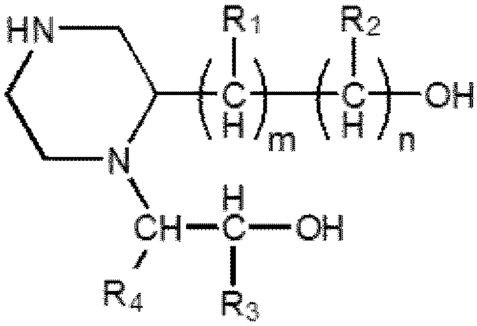

(1d)

[in the above formula (1d), $R_1$ to $R_4$ , m and n are the same as defined in the above formulae (6) and (7)], which is subjected to an intramolecular dehydration condensation reaction in the presence of an acid catalyst, to obtain a hydroxyalkyltriethylenediamine represented by the following formula (2b):

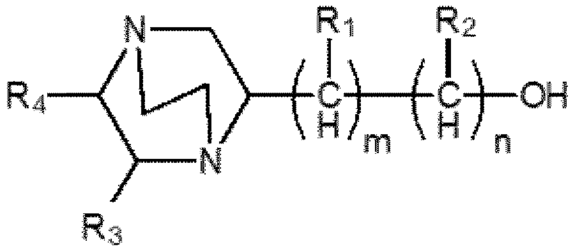

(2b)

[in the above formula (2b), $R_1$ to $R_4$ , m and n are the same as defined in the above formulae (6) and (7)] and/or a hydroxyalkyltriethylenediamine represented by the following formula (2c):

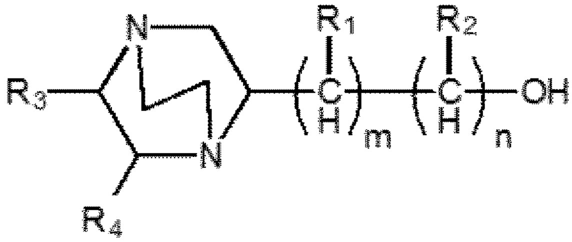

(2c)

[in the above formula (2c), $R_1$ to $R_4$ , m and n are the same as defined in the above formulae (6) and (7)].
[19] The process according to the above [18], wherein the alkylene oxide is ethylene oxide or propylene oxide.

(II) Process for producing a hydroxyalkylpiperazine and/or hydroxypiperazine not forming part of the invention :

[20] A process for producing a hydroxyalkylpiperazine and/or hydroxypiperazine represented by the formula (6) as defined in the above [18], which comprises subjecting a dihydroxyalkylethylenediamine represented by the following formula (8a):

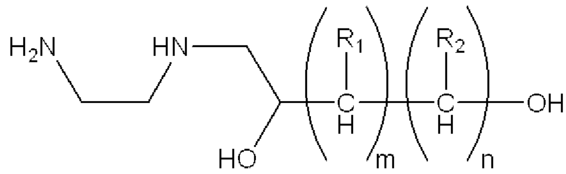

(8a)

[in the above formula (8a), $R_1$, $R_2$, m and n are the same as defined in the above formula (6)] and/or a dihydroxyalkylethylenediamine represented by the following formula (8b):

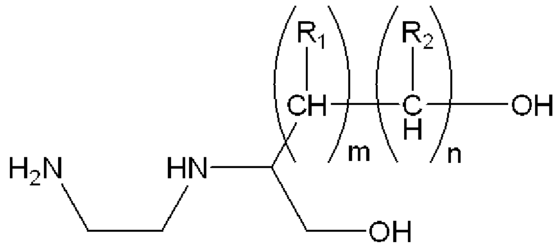

(8b)

[in the above formula (8b), $R_1$, $R_2$, m and n are the same as defined in the above formula (6)] to an intramolecular dehydration condensation reaction in the presence of an acid catalyst or a Raney metal catalyst.

[21] The process according to the above [20], wherein the acid catalyst comprises one or more compounds selected from the group consisting of a metal phosphate and an organic phosphorus compound.

[22] The process according to the above [20] or [21], wherein the Raney metal catalyst comprises a Raney copper catalyst.

(I-2) Process for producing a hydroxyalkyltriethylenediamine not forming part of the present invention:

[23] A process for producing a hydroxymethyltriethylenediamine represented by the following formula (2d):

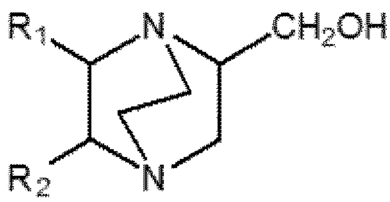

(2d)

[in the above formula (2d), $R_1$ and $R_2$ are the same as defined in the following formula (10)], which comprises subjecting a piperazine represented by the following formula (10):

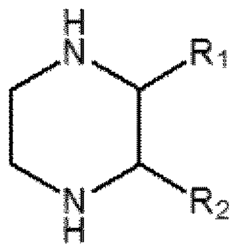

(10)

[in the above formula (10), each of $R_1$ and $R_2$ which are independent of each other, is a hydrogen atom or a $C_{1-4}$ alkyl group] and glycerol, to an intramolecular dehydration condensation reaction in the presence of an acid catalyst.

[24] The process according to the above [23], wherein the piperazine represented by the formula (10) is one or more piperazines selected from the group consisting of piperazine, methylpiperazine, ethylpiperazine and dimethylpiperazine.

[25] The process according to the above [23] or [24], wherein the acid catalyst comprises one or more compounds selected from the group consisting of a metal phosphate and an organic phosphorus compound.

ADVANTAGEOUS EFFECTS OF THE INVENTION

By the process for producing a hydroxyalkyltriethylenediamine or hydroxytriethylenediamine not forming part of the present invention, there will be no formation of a by-product salt, and the desired product can be obtained in one stage, whereby it is possible to obtain a hydroxyalkyltriethylenediamine or hydroxytriethylenediamine simply and in a small number of steps, as compared with the conventional processes.

Further, by a process wherein no reducing compound is employed, within the process for producing a hydroxyalkyltriethylenediamine or hydroxytriethylenediamine not forming part of the present invention, it is possible to obtain a hydroxyalkyltriethylenediamine simply and safely, as compared with the conventional processes.

Further, according to the process for producing a hydroxyalkylpiperazine and/or hydroxypiperazine not forming part of the present invention, the desired product can be obtained in one stage, and it is possible to obtain a hydroxyalkylpiperazine simply and efficiently as compared with the conventional processes.

Further, by a process of using an acid catalyst within the process for producing a hydroxyalkylpiperazine and/or hydroxypiperazine not forming part of the present invention, it is possible to obtain a hydroxyalkylpiperazine simply and safely as compared with the conventional processes, since hydrogen having a risk of catching fire and/or a reducing compound is not used.

By the catalyst composition for the production of a polyurethane resin of the present invention, and the process for producing a polyurethane resin, which uses the catalyst composition, it is possible to produce a polyurethane product with good productivity and moldability.

And, the polyurethane resin produced by using the catalyst composition of the present invention is substantially free from an amine emission from the polyurethane resin and thus is effective for preventing discoloration of PVC (vinyl chloride resin) of an instrument panel of an automobile attributable to a conventional tertiary amine compound or preventing a fogging phenomenon of a window glass due to migration of a volatile component from the polyurethane foam.

MODE FOR CARRYING OUT THE INVENTION

Now, the present invention will be described in detail.

Firstly, the process for producing a hydroxyalkyltriethylenediamine or hydroxytriethylenediamine not forming part of the present invention will be described.

The first process for producing a hydroxyalkyltriethylenediamine or hydroxytriethylenediamine (hereinafter sometimes referred to as "the first process") comprises subjecting a mono-substituted dihydroxyalkylpiperazine and/or a di-substituted hydroxyalkylpiperazine to an intramolecular dehydration condensation reaction in the presence of an acid catalyst.

In the above first process, the reaction is carried out by contacting the mono-substituted dihydroxyalkylpiperazine and/or the di-substituted hydroxyalkylpiperazine with the acid catalyst.

The acid catalyst may, for example, be a phosphorus-containing substance such as a metal phosphate or an organic phosphorus compound, a nitrogen-containing substance, a sulfur-containing substance, a niobium-containing substance, silica, alumina, silica-alumina, silica-titania, zeolite, heteropolyacid, a Group 4B metal oxide condensation catalyst, a Group 6B metal-containing condensation catalyst, a Brønsted acid, a Lewis acid or a phosphorus-containing amide. Among them, a phosphorus-containing substance is particularly preferred.

The above-mentioned metal phosphate may, for example, be a metal salt of phosphoric acid, phosphorous acid or hypophosphorous acid. The metal to form a salt with phosphoric acid is not particularly limited, but it may, for example, be sodium, potassium, lithium, calcium, barium, magnesium, aluminum, titanium, iron, cobalt, nickel, copper, zinc, zirconium, palladium, silver, tin or lead.

Further, the above-mentioned organic phosphorus compound may be a conventional one and is not particularly limited, and it may for example, be a phosphoric acid ester such as methyl phosphate; a phosphoric acid diester such as dimethyl phosphate; a phosphoric acid triester such as triphenyl phosphate; phosphorous acid; a phosphorous acid ester such as methyl phosphite or phenyl phosphite; a phosphorous acid diester such as diphenyl phosphite; a phosphorous acid triester such as triphenyl phosphite; an aryl phosphonic acid such as phenyl phosphonic acid; an alkyl phosphonic

acid such as methyl phosphonic acid; an alkyl phosphonic acid such as methyl phosphonic acid; an aryl phosphonic acid such as phenyl phosphonic acid; an alkyl phosphinic acid such as dimethyl phosphinic acid; an aryl phosphinic acid such as diphenyl phosphinic acid; an alkylaryl phosphinic acid such as phenylmethyl phosphinic acid; an alkyl phosphinic acid such as dimethyl phosphinic acid; an aryl phosphinic acid such as diphenyl phosphinic acid; an alkylaryl phosphinic acid such as phenylmethyl phosphinic acid; an acidic phosphoric acid ester such as lauryl acid phosphate, tridecyl acid phosphate or stearyl acid phosphate; or a salt of an acidic phosphoric acid ester.

In the above first process, one or more members selected from the above organic phosphorus compounds may be used.

The amount of the acid catalyst to be used in the first process is not particularly limited, but it is usually within a range of from 0.01 to 20 wt%, preferably within a range of from 0.1 to 10 wt%, based on the total amount of the mono-substituted dihydroxyalkylpiperazine and the di-substituted hydroxyalkylpiperazine, as the raw materials. If it is less than 0.01 wt%, the reaction tends to be substantially slow, and if it exceeds 20 wt%, such tends to lead to an economical disadvantage.

In the above first process, the reaction may be carried out in a gas phase or in a liquid phase. Further, the reaction may be carried out in a batch system, a semi-batch system or a continuous system, or in a fixed bed flow system. Industrially, a fixed bed flow system is advantageous from the viewpoint of the operation, apparatus and economical efficiency.

In the above first process, as a diluent, an inert gas such as nitrogen gas, hydrogen gas, ammonia gas, steam or a hydrocarbon, or an inert solvent such as water or an inert hydrocarbon, may be used to dilute the mono-substituted dihydroxyalkylpiperazine and/or the di-substituted hydroxyalkylpiperazine as the raw material thereby to facilitate the reaction. Such a diluent may be used in an optional amount, and although not limited thereto, the molar ratio of [total amount of the mono-substituted dihydroxyalkylpiperazine and the di-substituted hydroxyalkylpiperazine]/[the amount of the diluent] is preferably within a range of from 0.01 to 1, more preferably within a range of from 0.05 to 0.5. When the molar ratio is at least 0.01, the productivity of the hydroxyalkyltriethylenediamine or hydroxytriethylenediamine will be improved. On the other hand, when the molar ratio is at most 1, the selectivity for the hydroxyalkyltriethylenediamine or hydroxytriethylenediamine will be improved.

In the above first process, the diluent may be introduced into the reactor at the same time as the mono-substituted dihydroxyalkylpiperazine and/or the di-substituted hydroxyalkylpiperazine, or the mono-substituted dihydroxyalkylpiperazine and/or the di-substituted hydroxyalkylpiperazine is preliminarily dissolved in the diluent and then introduced in the form of a raw material solution into the reactor.

In the above first process, in a case where the reaction is carried out in a gas phase, it is usually carried out in the coexistence of a gas inert to the reaction such as nitrogen gas or argon gas. The amount of such an inert gas is not particularly limited, but it is usually within a range of from 1 to 20 mol, preferably from 2 to 10 mol, per mol of the total amount of the mono-substituted dihydroxyalkylpiperazine and the di-substituted hydroxyalkylpiperazine, as the raw materials.

In the above first process, the reaction temperature is usually within a range of from 150 to 500°C, preferably from 200 to 400°C. When it is at most 500°C, decomposition of the raw materials and the product can be suppressed, whereby the selectivity for the hydroxyalkyltriethylenediamine will be improved, and when it is at least 150°C, a sufficient reaction rate can be obtained.

In the above first process, in a case where the reaction is carried out in a gas phase, after the completion of the reaction, the reaction gas mixture containing the hydroxyalkyltriethylenediamine is dissolved in water or an acidic aqueous solution to obtain a reaction mixture solution containing the hydroxyalkyltriethylenediamine. And, from the obtained reaction mixture solution, the hydroxyalkyltriethylenediamine can be obtained by a desired separation purification operation such as extraction, concentration or the like. Otherwise, by means of a hydrohalic acid, it may be obtained as a hydrohalic acid salt.

In the above first process, for example, when a mono-substituted dihydroxyalkylpiperazine represented by the above formula (1a) or (1b) is subjected to an intramolecular dehydration condensation reaction in the presence of an acid catalyst, a hydroxyalkyltriethylenediamine or hydroxytriethylenediamine represented by the above formula (2a) can be obtained.

In the above formulae (1a), (1b) and (2a), R is a hydrogen atom or a linear or branched $C_{1-4}$ alkyl group, and specifically, a methyl group, an ethyl group, a propyl group, an isopropyl group or a butyl group may, for example, be mentioned. Among them, a methyl group or an ethyl group is preferred. Further, in the above formulae (1a), (1b) and (2a), n is an integer of from 0 to 6, preferably an integer of from 0 to 2.

The mono-substituted dihydroxyalkylpiperazine represented by the above formula (1a) is not particularly limited, but it may, for example, be a dihydroxypropylpiperazine, a dihydroxybutylpiperazine, a dihydroxypentylpiperazine or a dihydroxyhexylpiperazine. The mono-substituted dihydroxyalkylpiperazine represented by the above formula (1a) may specifically be a dihydroxypropylpiperazine represented by the following formula (3a):

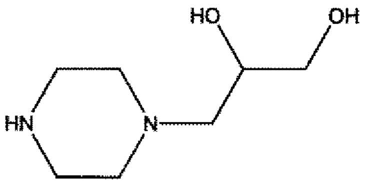

(3a)

The mono-substituted dihydroxyalkylpiperazine represented by the above formula (1b) is not particularly limited, but it may, for example, be a dihydroxypropylpiperazine, a dihydroxybutylpiperazine, a dihydroxypentylpiperazine or a dihydroxyhexylpiperazine. Specifically, the mono-substituted dihydroxyalkylpiperazine represented by the above formula (1b) may, for example, be dihydroxypropylpiperazine represented by the following formula (3b):

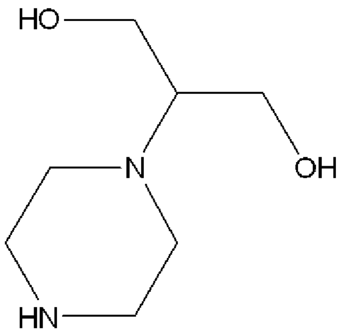

(3b)

In the above first process, the hydroxyalkyltriethylenediamine (where n=1 to 6) or hydroxytriethylenediamine (where n=0) represented by the above formula (2a) is not particularly limited, but it may, for example, be hydroxytriethylenediamine, hydroxymethyltriethylenediamine, hydroxyethyltriethylenediamine, hydroxypropyltriethylenediamine or hydroxybutyltriethylenediamine.

In the above first process, the mono-substituted dihydroxyalkylpiperazine represented by the above formula (1a) or (1b) is not particularly limited, and for example, a commercial product may be used, or a synthesized product may be used.

The mono-substituted dihydroxyalkylpiperazine represented by the above formula (1a) is not particularly limited, but it may, for example, be one obtained by an addition reaction of piperazine with a compound represented by the above formula (4a), or one obtained by a dehydration condensation reaction of piperazine with a compound represented by the above formula (4b) in the presence of an acid catalyst, or one obtained by a reaction of a piperazine with a compound represented by the above formula (4c).

Specifically, for example, the dihydroxypropylpiperazine represented by the above formula (3a) may be obtained by an addition reaction of piperazine with glycidol, or may be obtained by a dehydration condensation reaction of piperazine with glycerin in the presence of an acid catalyst. Further, also by reacting piperazine with chloropropanediol, dihydroxypropylpiperazine represented by the above formula (3a) can be obtained.

Here, as the acid catalyst, the above-described acid catalyst to be used at the time of the intramolecular dehydration condensation reaction of the mono-substituted dihydroxyalkylpiperazine represented by the above formula (1a) or (1b) may be used. For example, a phosphorus-containing substance such as a metal phosphate or an organic phosphorus compound, a nitrogen-containing substance, a sulfur-containing substance, a niobium-containing substance, silica, alumina, silica-alumina, silica-titania, zeolite, heteropolyacid, a Group 4B metal oxide condensation catalyst, a Group 6B metal-containing condensation catalyst, a Brønsted acid, a Lewis acid or a phosphorus-containing amide may, for example, be mentioned. Among them, a phosphorus-containing substance is particularly preferred.

Further, the mono-substituted dihydroxyalkylpiperazine represented by the above formula (1b) is not particularly limited, but it may, for example, be one obtained by reacting piperazine with a dihydroxyketone represented by the above formula (5a), followed by hydrogen reduction, or one obtained by reducing a dialkylester of piperazine obtained by using a reducing agent such as lithium aluminum hydride or sodium dihydro-bis(2-methoxyethoxy)aluminate after preparing a dialkylester of piperazine by reacting piperazine with a dialkyl halogenated dicarboxylate represented by the above formula (5b).

Specifically, for example, the dihydroxypropylpiperazine presented by the above formula (3b) can be obtained by reacting piperazine with dihydroxyacetone in the presence of a hydrogenation catalyst. Further, for example, the dihydroxypropylpiperazine represented by the above formula (3b) can be obtained also by a method wherein piperazine is reacted with diethyl bromomaleate to prepare a diethylester of piperazine, and then reducing the obtained diethylester of piperazine by means of a reducing agent such as lithium aluminum hydride or sodium dihydro-bis(2-methoxyethoxy)aluminate.

Further, in the above process, for example, a hydroxyalkylpiperazine or hydroxypiperazine represented by the following formula (6):

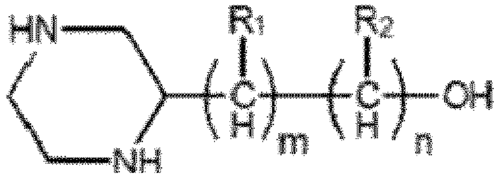

(6)

[in the above formula (6), each of $R_1$ and $R_2$ which are independent of each other, is a hydrogen atom or a linear or branched $C_{1-4}$ alkyl group, and each of m and n which are independent of each other, is an integer of from 0 to 2, provided m+n<4], is reacted with an alkylene oxide represented by the following formula (7):

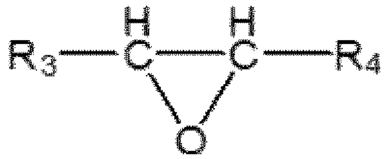

(7)

[in the above formula (7), each of $R_3$ and $R_4$ which are independent of each other, is a hydrogen atom or a linear or branched $C_{1-4}$ alkyl group] to obtain a di-substituted hydroxyalkylpiperazine represented by the following formula (1c):

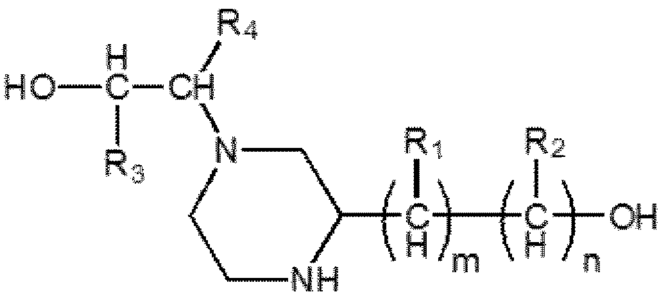

(1c)

[in the above formula (1c), $R_1$ to $R_4$, m and n are the same as defined in the above formulae (6) and (7)] and/or a di-substituted hydroxyalkylpiperazine represented by the following formula (1d):

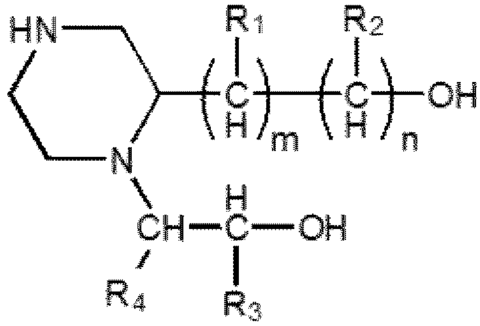

(1d)

[in the above formula (1d), $R_1$ to $R_4$, m and n are the same as defined in the above formulae (6) and (7)], which is subjected to an intramolecular dehydration condensation reaction in the presence of an acid catalyst, to obtain a hydroxyalkyltriethylenediamine represented by the following formula (2b):

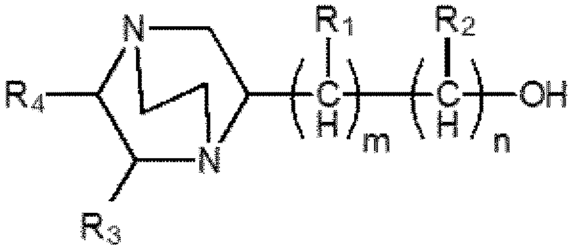

(2b)

[in the above formula (2b), $R_1$ to $R_4$, m and n are the same as defined in the above formulae (6) and (7)] and/or a hydroxyalkyltriethylenediamine represented by the following formula (2c):

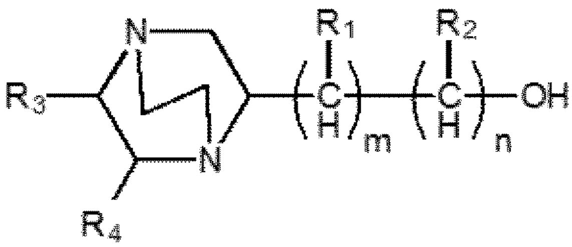

(2c)

[in the above formula (2c), $R_1$ to $R_4$, m and n are the same as defined in the above formulae (6) and (7)].

In the above formulae (6), (1c), (1d), (2b) and (2c), each of substituents $R_1$ and $R_2$ which are independent of each other, is a hydrogen atom or a linear or branched $C_{1-4}$ alkyl group, and specifically, a methyl group, an ethyl group, a propyl group, an isopropyl group or a butyl group may, for example, be mentioned. Among them, a hydrogen atom, a methyl group or an ethyl group is preferred. Further, in the above formulae (6), (1c), (1d), (2b) and (2c), each of m and n which are independent of each other, is an integer of from 0 to 2.

In the above formulae (7), (1c), (1d), (2b) and (2c), each of substituents $R_3$ and $R_4$ which are independent of each other, is a hydrogen atom or a linear or branched $C_{1-4}$ alkyl group, and specifically, a methyl group, an ethyl group, a propyl group, an isopropyl group or a butyl group may, for example, be mentioned. Among them, a hydrogen atom, a methyl group or an ethyl group is preferred.

The hydroxyalkylpiperazine represented by the above formula (6) is not particularly limited, but it may, for example, be 2-hydroxymethylpiperazine, 2-hydroxyethylpiperazine, 2-(hydroxypropyl)piperazine, hydroxybutylpiperazine, hydroxypentylpiperazine or hydroxyhexylpiperazine.

The hydroxyalkylpiperazine represented by the above formula (6) to be used in the above first process may, for example, be one obtained by reacting an ethylenediamine derivative with diethyl bromomalonate, followed by reduction for deprotection (J. Med. Chem. 36, 2075 (1993)). Otherwise, one obtained by reducing a piperazinecarboxylic acid hydrochloride in the presence of a catalyst may be used. Further, one obtained by subjecting a dihydroxyalkylethylenediamine to an intramolecular dehydration condensation reaction in the presence of an acid catalyst or a Raney metal catalyst, may be used (this method will be described hereinafter).

Further, the alkylene oxide represented by the above formula (7) to be used in the above first process, is not particularly limited, but for example, ethylene oxide or propylene oxide may be mentioned as preferred.

In the above first process, the hydroxyalkylpiperazine represented by the above formula (6) is reacted with an alkylene oxide represented by the above formula (7) to obtain a di-substituted hydroxyalkylpiperazine represented by the above formula (1c) and/or a di-substituted hydroxyalkylpiperazine represented by the formula (1d).

The obtainable di-substituted hydroxyalkylpiperazine is not particularly limited, but it may, for example, be 1-hydroxyethyl-3-hydroxymethylpiperazine or 1-(1'-methyl-2'-hydroxyethyl)-3-hydroxymethylpiperazine.

In this process, the di-substituted hydroxyalkylpiperazine thus obtained is subjected to an intramolecular dehydration condensation reaction in the presence of an acid catalyst to obtain a hydroxyalkyltriethylenediamine represented by the above formula (2b) and/or a hydroxyalkyltriethylenediamine represented by the above formula (2c).

The obtainable hydroxyalkyltriethylenediamine is not particularly limited, but it may, for example, be 2-hydroxymethyltriethylenediamine, 2-hydroxymethyl-6-methyltriethylenediamine, 2-hydroxyethyltriethylenediamine, hydroxypropyltriethylenediamine or hydroxybutyltriethylenediamine.

Now, a process for producing a hydroxyalkylpiperazine and/or hydroxypiperazine not forming part of the present invention will be described.

According to this process, it is possible to produce a hydroxyalkylpiperazine represented by the above formula (6)

to be used as a raw material in the above first process.

The process for producing a hydroxyalkylpiperazine and/or hydroxypiperazine not forming part of the present invention comprises subjecting a dihydroxyalkylenediamine represented by the above formula (8a) and/or a dihydroxyalkylethylenediamine represented by the above formula (8b) to an intramolecular dehydration condensation reaction in the presence of an acid catalyst or a Raney metal catalyst to obtain a hydroxyalkylpiperazine represented by the above formula (6).

In the above process, substituents $R_1$, $R_2$, m and n in the above formulae (8a) and (8b) are the same as defined in the above formula (6).

In the above process, the dihydroxyalkylethylenediamine to be used may be a compound represented by the above formula (8a) or (8b) and is not particularly limited. For example, it may be a dihydroxypropylethylenediamine, a dihydroxybutylethylenediamine, a dihydroxypentylethylenediamine or a dihydroxyhexylethylenediamine.

In the above process, as such a dihydroxyalkylethylenediamine, a commercial product may be used, or a synthesized product may be used.

In the above process, the dihydroxyalkylethylenediamine represented by the above formula (8a) may specifically be, for example, a dihydroxypropylethylenediamine represented by the following formula (9a):

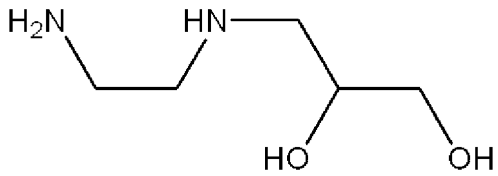

(9a)

This dihydroxypropylethylenediamine may, for example, be obtained by an addition reaction of ethylenediamine with an epoxy alcohol such as glycidol, or by an addition reaction of ethylenediamine with chloropropanediol.

Further, it may also be obtained by reacting ethylenediamine with a dihydroxyketone, followed by hydrogen reduction. Further, the dihydroxypropylethylenediamine represented by the above formula (9a) may be obtained also by reacting ethylenediamine with a dialkyl halogenated dicarboxylate to obtain a diethylester of ethylenediamine, and then, the obtained diethylester of ethylenediamine is reduced by means of a reducing agent such as lithium aluminum hydride or sodium dihydro-bis(2-methoxyethoxy)aluminate.

In the above process, the dihydroxyalkylethylenediamine represented by the above formula (8b) may specifically be, for example, a dihydroxypropylethylenediamine represented by the following formula (9b):

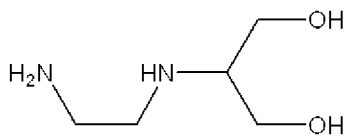

(9b)

This dihydroxypropylethylenediamine may, for example, be obtained by reacting ethylenediamine with dihydroxyacetone in the presence of a hydrogenation catalyst.

Otherwise, it is possible to obtain the dihydroxypropylpiperazine represented by the above formula (9b) also by a method wherein ethylenediamine and a dibromopropionic acid ester are reacted to prepare an ethylester or ethylenediamine, and then, the obtained ethylester of ethylenediamine is reduced by means of a reducing agent such as lithium aluminum hydride or sodium dihydro-bis(2-methoxyethoxy)aluminate.

The hydroxyalkylpiperazine represented by the above formula (6) obtained by the above process is not particularly limited, but it may, for example, be hydroxypiperazine, hydroxymethylpiperazine, hydroxyethylpiperazine, hydroxypropylpiperazine or hydroxybutylpiperazine. In the above process, the intramolecular dehydration condensation reaction is carried out by contacting the dihydroxyalkylethylenediamine represented by the above formula (8a) and/or the dihydroxyalkylethylenediamine represented by the above formula (8b) with an acid catalyst or a Raney metal catalyst.

In the above process, the acid catalyst may, for example, be a phosphorus-containing substance such as a metal phosphate or an organic phosphorus compound, a nitrogen-containing substance, a sulfur-containing substance, a niobium-containing substance, silica, alumina, silica-alumina, silica-titania, zeolite, heteropolyacid, a Group 4B metal oxide condensation catalyst, a Group 6B metal-containing condensation catalyst, a Bronsted acid, a Lewis acid or a phosphorus-containing amide. Among them, a phosphorus-containing substance is particularly preferred.

The above metal phosphate may be a conventional one and is not particularly limited, but for example, a metal salt of phosphoric acid, phosphorous acid or hypophosphorous acid may be mentioned. The metal to form a salt with phosphoric acid may, for example, be sodium, potassium, lithium, calcium, barium, magnesium, aluminum, titanium, iron, cobalt, nickel, copper, zinc, zirconium, palladium, silver, tin or lead.

The above organic phosphorus compound is not particularly limited and may be a conventional one, and it is the same as exemplified in the above first process.

In the above process, one or more selected from these compounds may be used as the acid catalyst.

In the above process, the amount of the acid catalyst to be used is not particularly limited, but it is usually within a range of from 0.01 to 20 wt%, preferably within a range of from 0.1 to 10 wt%, based on the total amount of the dihydroxyalkylethylenediamine represented by the above formula (8a) and the dihydroxyalkylethylenediamine represented by the above formula (8b), as the raw materials. If it is less than 0.01 wt%, the reaction tends to be remarkably slow, and if it exceeds 20 wt%, such tends to be economically disadvantageous.

Further, in the above process, the Raney metal catalyst may, for example, be a Raney copper catalyst, a Raney nickel catalyst, a Raney cobalt catalyst or a Raney iron catalyst. In the above process for producing a hydroxyalkylpiperazine, it is possible to employ one or more selected from the above Raney metal catalysts. However, in order to improve the yield of the desired product, a Raney copper catalyst may particularly suitably be used. Further, in the above process for producing a hydroxyalkylpiperazine, a synthesized product or a commercial product may be used as the Raney metal catalyst.

The Raney metal catalyst to be used in the above process, may contain an optional catalytically active metal within a range not to depart from the concept of the present invention.

In the above process for producing a hydroxyalkylpiperazine, the amount of the Raney metal catalyst is not particularly limited, but it is usually within a range of from 0.1 to 20 wt%, preferably within a range of from 0.5 to 10 wt%, based on the total amount of the dihydroxyalkylethylenediamine represented by the above formula (8a) and the dihydroxyalkylethylenediamine represented by the above formula (8b) as the raw materials. If it is less than 0.1 wt%, the reaction tends to be remarkably slow, and if it exceeds 20 wt%, such tends to be economically disadvantageous.

In the above process, the reaction may be carried out in a gas phase or in a liquid phase. Further, the reaction can be carried out in a batch system, a semi-batch system or a continuous system by a suspension bed or in a fixed bed flow system, but industrially, a fixed bed flow system is advantageous from the viewpoint of the operation, apparatus and economical efficiency. In the above process, as a diluent, an inert gas such as nitrogen gas, hydrogen gas, ammonia gas, steam or a hydrocarbon, or an inert solvent such as water or an inert hydrocarbon may be used to dilute the dihydroxyalkylethylenediamine represented by the above formula (8a) or (8b) as the raw material thereby to facilitate the reaction. Such a diluent can be used in an optional amount, and although not limited, the molar ratio of [total amount of the dihydroxyalkylethylenediamine represented by the above formula (8a) and the dihydroxyalkylethylenediamine represented by the above formula (8b)]/[amount of the diluent] is preferably within a range of from 0.01 to 1. When the molar ratio is at least 0.01, the productivity of the hydroxyalkylpiperazine represented by the above formula (6) will be improved. On the other hand, when the molar ratio is at most 1, the selectivity for the hydroxyalkylpiperazine represented by the above formula (6) will be improved.

In the above process, the diluent may be introduced into the reactor at the same time as the hydroxyalkylethylenediamine represented by the above formula (8a) or (8b), or the dihydroxyalkylethylenediamine represented by the above formula (8a) or (8b) may be preliminarily dissolved in the diluent and then introduced in the form of the raw material solution into the reactor.

In the above process, in a case where the reaction is carried in a gas phase, it is usually carried out in the coexistence of a gas inert to the reaction, such as nitrogen gas or argon gas. The amount of such a gas is not particularly limited, but it is usually within a range of from 1 to 20 mol, preferably from 2 to 10 mol, per mol of the total amount of the dihydroxyalkylethylenediamine represented by the above formula (8a) and the dihydroxyalkylethylenediamine represented by the above formula (8b), as the raw materials.

In the above process, the reaction temperature in a case where an acid catalyst is used, is usually within a range of from 100 to 400°C, preferably from 150 to 300°C. When the reaction temperature is at most 400°C, decomposition of the raw materials and the product will be suppressed, whereby the selectivity for the hydroxyalkylpiperazine represented by the above formula (6) will be improved, and when it is at least 150°C, a sufficient reaction rate can be obtained. Further, the reaction temperature in a case where a Raney metal catalyst is used, is usually within a range of from 50 to 250°C, preferably from 100 to 200°C. When the reaction temperature is at most 250°C, decomposition of the raw materials and the product will be suppressed, whereby the selectivity for the hydroxyalkylpiperazine will be improved, and when it is at least 50°C, a sufficient reaction rate can be obtained.

In the above process, in a case where the reaction is carried out in a gas phase, after completion of the reaction, the reaction gas mixture containing the hydroxyalkylpiperazine represented by the above formula (6) is dissolved in water or an acidic aqueous solution to obtain a reaction mixture solution containing the hydroxyalkylpiperazine represented by the above formula (6). And, it is possible to obtain the hydroxyalkylpiperazine represented by the above formula (6) from the obtained reaction mixture solution by a desired separation purification operation such as extraction or concentration. Otherwise, by means of a hydrohalic acid, it can be obtained as a hydrohalic acid salt.

Now, the second process for producing a hydroxyalkyltriethylenediamine not forming part of the present invention (hereinafter sometimes referred to as "the second process") will be described.

The second process not forming part of the present invention comprises subjecting a piperazine represented by the above formula (10) and glycerin to an intermolecular dehydration condensation reaction in the presence of an acid catalyst to obtain a hydroxymethyltriethylenediamine represented by the above formula (2d).

Here, the piperazine represented by the above formula (10) may, for example, be piperazine, methylpiperazine, ethylpiperazine or dimethylpiperazine, as preferred. One of them may be used alone, or two or more of them may be used in combination.

As the acid catalyst, an acid catalyst to be used at the time of the intramolecular dehydration condensation reaction of the dihydroxyalkylpiperazine represented by the above formula (1a) or (1b) may be used. It may, for example, be a phosphorus-containing substance such as a metal phosphate or an organic phosphorus compound, a nitrogen-containing substance, a sulfur-containing substance, a niobium-containing substance, silica, alumina, silica-alumina, silica-titania, zeolite, heteropolyacid, a Group 4B metal oxide condensation catalyst, a Group 6B metal-containing condensation catalyst, a Bronsted acid, a Lewis acid or a phosphorus-containing amide. Among them, a phosphorus-containing substance such as a metal phosphate or an organic phosphorus compound is preferred.

In the above second process, the above metal phosphate may, for example, be a metal salt of phosphoric acid, phosphorous acid, hypophosphorous acid or the like. The metal to form a salt with phosphoric acid is not particularly limited, but it may, for example, be sodium, potassium, lithium, calcium, barium, magnesium, aluminum, titanium, iron, cobalt, nickel, copper, zinc, zirconium, palladium, silver, tin or lead.

Further, the above organic phosphorus compound is not particularly limited and may be a conventional one, and it is the same as one exemplified in the above first process.

In the above second process, one or more selected from the above-mentioned organic phosphorus compounds may be used.

In the above second process, the reaction may be carried out in a gas phase or in a liquid phase. Further, the reaction may be carried out in a batch system, a semi-batch system or a continuous system by a suspension bed or in a fixed bed flow system, but industrially, a fixed bed flow system is advantageous from the viewpoint of the operation, apparatus and economical efficiency.

In the above second process, as a diluent, an inert gas such as nitrogen gas, hydrogen gas, ammonia gas, steam or a hydrocarbon, or an inert solvent such as water or an inert hydrocarbon may be used to dilute the piperazine represented by the above formula (10) and/or glycerin as the raw material thereby to facilitate the above reaction. Such a diluent may be used in an optional amount and is not particularly limited, but the molar ratio of [the piperazine represented by the above formula (10)]/[the diluent], or the molar ratio of [glycerin]/[diluent] is preferably within a range of from 0.01 to 1, more preferably within a range of from 0.05 to 0.5. When the molar ratio is at least 0.01, the productivity of the hydroxymethyltriethylenediamine represented by the above formula (2d) will be improved. On the other hand, when the molar ratio is at most 1, the selectivity for the hydroxymethyltriethylenediamine represented by the above formula (2d) will be improved.

In the above second process, the above diluent may be introduced into the reactor at the same time as the piperazine represented by the above formula (10) and/or glycerin, or the piperazine represented by the above formula (10) and/or glycerin is preliminarily dissolved in the diluent and then introduced in the form of a raw material solution into the reactor.

In the above second process, in a case where the reaction is carried out in a gas phase, it is usually carried out in the coexistence of a gas inert to the reaction such as nitrogen gas or argon gas. The amount of such a gas to be used is usually within a range of from 1 to 20 mol, preferably from 2 to 10 mol, per mol of the piperazine represented by the above formula (10).

In the above second process, the molar ratio of [the piperazine represented by the above formula (10)]/[glycerin] is usually within a range of from 0.02 to 50, preferably from 0.05 to 20. When the molar ratio is at least 0.02 and at most 50, a side reaction will be suppressed, whereby the selectivity for the hydroxymethyltriethylenediamine represented by the above formula (2d) will be improved.

In the above second process, the reaction temperature is usually within a range of from 150 to 500°C, preferably from 200 to 400°C. When the reaction temperature is at most 500°C, decomposition of the raw materials and the product will be suppressed, whereby the selectivity for the hydroxymethyltriethylenediamine represented by the above formula (2d) will be improved, and when it is at least 150°C, a sufficient reaction rate can be obtained.

In the above second process, in a case where the reaction is carried out in a gas phase, after completion of the reaction, the reaction gas mixture containing the hydroxymethyltriethylenediamine represented by the above formula (2d) is dissolved through water or an acidic aqueous solution to obtain a reaction mixture solution containing the hydroxymethyltriethylenediamine represented by the above formula (2d). And, it is possible to obtain the hydroxymethyltriethylenediamine represented by the above formula (2d) from the obtained reaction mixture by a desired separation purification operation such as extraction or concentration. Otherwise, by means of a hydrohalic acid, it may be obtained as a hydrohalic acid salt.

Now, the catalyst composition for the production of a polyurethane resin of the present invention will be described.

The catalyst composition for the production of a polyurethane resin of the present invention comprises a hydroxyalkyltriethylenediamine or hydroxytriethylenediamine (A), and an amine compound (B) having, in its molecule, one or more substituents selected from the group consisting of a hydroxy group, a primary amino group and a secondary amino group, or, in a non-claimed embodiment, a tertiary amine compound (C) having a value of [blowing reaction rate constant/gelling reaction rate constant] of at least 0.5.

In the above catalyst composition, the above hydroxyalkyltriethylenediamine or hydroxytriethylenediamine (A) may, for example, be the hydroxyalkyltriethylenediamine or hydroxytriethylenediamine represented by the above formula (2a), the hydroxyalkyltriethylenediamine represented by the above formula (2b) and/or the hydroxyalkyltriethylenediamine represented by the above formula (2c), or the hydroxymethyltriethylenediamine represented by the above formula (2d). Among them, the hydroxymethyltriethylenediamine represented by the above formula (2d) is preferably employed. In the catalyst composition of the present invention, one of them may be used alone or two or more of them may be used in combination.

Further, in the above catalyst composition, the amine compound represented by the above formula (2e) may, for example, be hydroxytriethylenediamine, hydroxymethyltriethylenediamine or hydroxyethyltriethylenediamine, but 2-hydroxymethyltriethylenediamine is preferred, since it is industrially readily available.

The hydroxyalkyltriethylenediamine or hydroxytriethylenediamine represented by the above formulae (2a) to (2e) can be produced by the above-mentioned process of the present invention. Otherwise, the compound represented by the above formula (2e) can be produced also by a known method. For example, it can be produced by reacting piperazine with a corresponding dibromocarboxylic acid ester in a proper molar ratio and reducing the obtained ester.

In the above catalyst composition, the amine compound (B) having, in its molecule, one or more substituents selected from the group consisting of a hydroxy group, a primary amino group and a secondary amino group, is not particularly limited, but is preferably an amine compound represented by the above formula (11).

Each of the substituents $R_1$ to $R_8$ in the amine compound represented by the above formula (11), which are independent of one another, is preferably a hydrogen atom, a hydroxy group, a methyl group, a hydroxyethyl group, a hydroxypropyl group, an aminoethyl group, an aminopropyl group, a monomethylaminoethyl group, a monomethylaminopropyl group, a dimethylaminoethyl group or a dimethylaminopropyl group.

Specifically, the amine compound represented by the above formula (11) may, for example, be a primary amine compound such as N,N-dimethylethylenediamine, N,N-dimethylpropylenediamine, N,N-dimethyltetramethylenediamine, N,N-dimethylpentamethylenediamine, N,N-dimethylhexamethylenediamine, N,N-dimethylheptamethylenediamine, N,N-dimethyloctamethylenediamine, N,N-dimethylnonamethylenediamine, N,N-dimethyldecamethylenediamine, N-methylethylenediamine, N-methylpropylenediamine, N-methyltetramethylenediamine, N-methylpentamethylenediamine, N-methylhexamethylenediamine, N-methylheptamethylenediamine, N-methyloctamethylenediamine, N-methylnonamethylenediamine, N-methyldecamethylenediamine, N-acetylethylenediamine, N-acetylpropylenediamine, N-acetyltetramethylenediamine, N-acetylpentamethylenediamine, N-acetylhexamethylenediamine, N-acetylheptamethylenediamine, N-acetyloctamethylenediamine, N-acetylnonamethylenediamine, N-acetyldecamethylenediamine, N,N,N'-trimethyldiethylenetriamine, N,N,N',N"-tetramethyltriethylenetetramine, N,N,N',N",N"'-pentamethyltetraethylenepentamine or N,N,N',N",N"',N""-hexamethylpentaethylenehexamine;

a secondary amine compound such as N,N'-dimethylethylenediamine, N,N'-dimethylpropylenediamine, N,N'-dimethylhexamethylenediamine, trimethylethylenediamine, trimethylpropylenediamine, trimethyltetramethylenediamine, trimethylpentamethylenediamine, trimethylhexamethylenediamine, trimethylheptamethylenediamine, trimethyloctamethylenediamine, trimethylnonamethylenediamine, trimethyldecamethylenediamine, tetramethyldiethylenetriamine, pentamethyltriethylenetetramine, hexamethyltetraethylenepentamine, heptamethylpentaethylenehexamine, bis(N,N-dimethylaminopropyl)amine or N-methylpiperazine; or

an alkanolamine such as N,N-dimethylaminoethanol, N,N-dimethylaminoisopropanol, N,N-dimethylaminoethoxyethanol, N,N-dimethylaminoethoxyisopropanol, N,N,N'-trimethylaminoethylethanolamine, N,N-dimethylaminoethyl-N'-methylaminoethyl-N"-methylaminoethanol, N,N-dimethylaminoethyl-N'-methylaminoethyl-N"-methylaminoisopropanol, N,N-dimethylaminoethoxyethoxyethanol, N,N-dimethylaminoethoxyethoxyisopropanol, N,N-dimethyl-N'-(2-hydroxyethyl)ethylenediamine, N,N-dimethyl-N'-(2-hydroxyethyl)propanediamine, N,N-dimethyl-N',N'-bis(2-hydroxypropyl)-1,3-propanediamine, N,N,N'-trimethyl-N'-(2-hydroxyethyl)bis(2-aminoethyl)ether, N,N,N'-trimethyl-N'-(2-hydroxyisopropyl)bis(2-aminoethyl)ether, N,N-bis(3-dimethylaminopropyl)-N-isopropanolamine, N,N-dimethylaminohexanol, 5-dimethylamino-3-methyl-1-pentanol, N,N,N'-trimethyl-N'-(2-hydroxyethyl)propylenediamine or N,N,N'-trimethyl-N'-(2-hydroxypropyl)propylenediamine.

Among these amine compounds, particularly preferred from the viewpoint of high catalytic activities and according to the claimed invention is one or more amine compounds selected from the group consisting of N,N-dimethylethylenediamine, N,N'-dimethylethylenediamine, N,N-dimethylpropylenediamine, N,N'-dimethylpropylenediamine, N,N-dimethylhexamethylenediamine, N,N'-dimethylhexamethylenediamine, trimethyldiethylenetriamine, trimethylethylenediamine, trimethylpropylenediamine, trimethylhexamethylenediamine, tetramethyldiethylenetriamine, N,N-dimethylaminoethanol, N,N-dimethylaminoisopropanol, bis(3-dimethylaminopropyl)amine, N-methylpiperazine, N,N-dimethylaminoethoxyethanol, N,N,N'-trimethylaminoethylethanolamine, N,N-dimethylaminoethyl-N'-methylaminoethyl-N"-methylaminoisopropanol, N,N-dimethylaminoethoxyethoxyethanol, N,N-dimethyl-N',N'-bis(2-hydroxypropyl)-1,3-propanediamine, N,N,N'-trimethyl-N'-(2-hydroxyethyl)bis(2-aminoethyl) ether, N,N-bis(3-dimethylaminopropyl)-N-isopropanolamine, N,N-dimethylaminohexanol and N,N,N'-trimethyl-N'-(2-hydroxyethyl)propylenediamine.

The amine compound represented by the above formula (11) to be used in the above catalyst composition can easily be prepared by a known method. For example, a method by means of a reaction of a diol with a diamine or amination of an alcohol, a method by means of reduction-methylation of a monoaminoalcohol or diamine, a method by means of a reaction of an amine compound with an alkylene oxide, etc. may be mentioned.

In the above catalyst composition, the mixing ratio of the hydroxyalkyltriethylenediamine or hydroxytriethylenediamine (A) to the amine compound (B) having, in its molecule, one or more substituents selected from the group consisting of a hydroxy group, a primary amino group and a secondary amino group, is not particularly limited, but the mixing ratio is usually adjusted so that the weight ratio of the hydroxyalkyltriethylenediamine or hydroxyethylenediamine (A) to the amine compound having, in its molecule, one or more substituents selected from the group consisting of a hydroxy group, a primary amino group and a secondary amino group (i.e. [hydroxyalkyltriethylenediamine or hydroxytriethylenediamine (A)]/[amine compound (B) having, in its molecular, one or more substituents selected from the group consisting of a hydroxy group, a primary amino group and a secondary amino group]) becomes usually within a range of from 1/99 to 99/1, preferably within a range of from 5/95 to 95/5. If the weight ratio exceeds this range, the synergistic effect of both catalysts may not sometimes be obtainable, and there may be a case where no adequate performance is obtainable with respect to the catalytic activities and the physical properties of the polyurethane resin.

Further, in the above catalyst composition, the mixing ratio of the amine compound represented by the above formula (2e) to the amine compound represented by the above formula (11) is not particularly limited, but the mixing ratio is usually adjusted so that the weight ratio of the amine compound represented by the above formula (2e) to the amine compound represented by the above formula (11) (i.e. [amine compound represented by the above formula (2e)]/[amine compound represented by the above formula (11)]) becomes within a range of from 1/99 to 99/1, preferably within a range of from 5/95 to 95/5. If the weight ratio exceeds this range, the synergistic effect of both catalysts may not sometimes be obtainable, and there may be case where no adequate performance is obtainable with respect to the catalytic activities and the physical properties of the polyurethane resin.

The tertiary amine compound (C) not forming part of the claimed invention having a value of [blowing reaction rate constant/gelling reaction rate constant] of at least 0.5, to be used in the above catalyst composition, is not particularly limited, but it may, for example, be triethanolamine, bis(2-dimethylaminoethyl) ether, N,N,N',N",N"-pentamethyldiethylenetriamine, hexamethyltriethylenetetramine, N,N-dimethylaminoethoxyethanol, N,N,N'-trimethylaminoethylethanolamine, N,N-dimethylaminoethyl-N'-methylaminoethyl-N"-methylaminoisopropanol or N,N,N'-trimethyl-N'-(2-hydroxyethyl)bis(2-aminoethyl)ether.

In the above catalyst composition, the gelling reaction rate constant (k1w) is a parameter calculated by the following method.

That is, toluene diisocyanate and diethylene glycol are charged so that the molar ratio of isocyanate group/hydroxy group becomes 1.0, and a predetermined amount of a tertiary amine compound is added as a catalyst, and a reaction is carried out by maintaining the temperature at a constant level in a benzene solvent, whereupon the amount of non-reacted isocyanate is measured. Here, when the reaction of toluene diisocyanate with diethylene glycol is assumed to be linear to the respective concentrations, the following formula will be established.

$$dx/dt=k(a-x)^2 \qquad (1)$$

In the above formula (1),

x: concentration of reacted NCO groups (mol/L),
a: initial concentration of NCO groups (mol/L),
k: reaction rate constant (L/mol·h),
t: reaction time (h).

When the initial conditions of t=0 and x=0 are substituted into the above formula (1), followed by integration, the following formula will be established.

$$1/(a-x)=kt+1/a \qquad (2)$$

From the above formula (2), the reaction rate constant k is obtained and substituted into the following formula (3) to obtain the catalyst constant Kc.

$$k=ko+KcC \qquad (3)$$

In the above formula (3),

ko: reaction rate constant in the absence of catalyst (L/mol·h),
Kc: catalyst constant ($L^2$/g·mol·h),
C: catalyst concentration in the reaction system (mol/L).

The obtained catalyst constant Kc is divided by the molecular weight (mc) of the catalyst to obtain the gelling reaction rate constant k1w ($L^2$/g·mol·h) which can be regarded as an activity power per weight (the following formula).

$$Kc/mc=k1w \qquad (4)$$

On the other hand, the blowing reaction constant (k2w) of the tertiary amine compound is obtained in the same manner as described above by reacting the toluene diisocyanate with water in a benzene solvent under the same conditions as in the above-described gelling reaction.

$$Kc/mc=k2w \qquad (5)$$

The tertiary amine compound to be used in the above catalyst composition can easily be prepared by a method known in literatures. For example, a method by means of a reaction of a diol with a diamine or amination of an alcohol, a method by means of reduction methylation of a monoaminoalcohol or diamine, a method by means of a reaction of an amine compound with an alkylene oxide, etc. may be mentioned.

In the above catalyst composition not forming part of the claimed invention, the mixing ratio of the hydroxyalkyltriethylenediamine or hydroxytriethylenediamine (A) to the tertiary amine compound (C) having a value of [blowing reaction rate constant/gelling reaction rate constant] of at least 0.5, is not particularly limited, but the mixing ratio is usually adjusted so that the weight ratio of the hydroxyalkyltriethylenediamine or hydroxytriethylenediamine (A) to the tertiary amine compound (C) having a value of [blowing reaction rate constant/gelling reaction rate constant] of at least 0.5 (i.e. [the above amine compound (A)]/[the above tertiary amine compound (C)]) becomes usually within a range of from 1/30 to 30/1, preferably within a range of from 1/20 to 20/1. If the weight ratio exceeds this range, the synergistic effect of both catalysts may not sometimes be obtainable, and there will be a case where no adequate performance is obtainable with respect to the catalytic activities and the physical properties of the polyurethane resin.

In the above catalyst composition, the hydroxyalkyltriethylenediamine or hydroxytriethylenediamine (A) and the amine compound (B) having, in its molecule, one or more substituents selected from the group consisting of a hydroxy group, a primary amino group and a secondary amino group according to the claimed invention, or the tertiary amine compound (C) not forming part of the claimed invention having a value of [blowing reaction rate constant/gelling reaction rate constant) of at least 0.5 to be used as the catalyst composition, may be preliminarily mixed, and such a mixture may be added at the time of the reaction, or they may be added simultaneously at the time of the reaction. Further, when they are mixed, they may be used as dissolved in a solvent. Such a solvent is not particularly limited, but it may, for example, be an organic solvent, such as an alcohol such as an ethylene glycol, diethylene glycol, dipropylene glycol, propylene glycol, butanediol or 2-methyl-1,3-propanediol, a hydrocarbon such as toluene, xylene, mineral terpene or mineral spirit, an ester such as ethyl acetate, butyl acetate, methylene glycol acetate or acetic acid cellosolve, a ketone such as methyl ethyl ketone, methyl isobutyl ketone or cyclohexanone, an amide such as N,N-dimethylformamide or N,N-dimethylacetamide; a chelating solvent, such as a β-diketone such as acetylacetone or its fluorinated substituted product, or a ketoester such as methyl acetoacetate or ethyl acetoacetate; or water.

Now, the process for producing a polyurethane resin of the present invention will be described.

The process for producing a polyurethane resin of the present invention comprises reacting a polyol with an isocyanate in the presence of the above-described catalyst composition of the present invention and, if necessary, a blowing agent, a surfactant, a flame retardant, a crosslinking agent, etc.

In the above process for producing a polyurethane resin, the amount of the catalyst composition of the present invention to be used is usually within a range of from 0.01 to 30 parts by weight, preferably within a range of from 0.1 to 20 parts by weight, per 100 parts by weight of the polyol to be used. If it is less than 0.01 part by weight, there may be case where no effect of the catalyst is obtainable. On the other hand, if it exceeds 30 parts by weight, not only an additional effect for the increase of the catalyst tends to be obtainable, but also the physical properties of the polyurethane resin may

sometimes deteriorate.

In the above process for producing a polyurethane resin, the polyol to be used may, for example, be a conventional polyether polyol, polyester polyol, polymer polyol or a flame-resistant polyol such as a phosphorus-containing polyol or a halogen-containing polyol. These polyols may be used alone or in combination as a mixture.

The polyether polyol to be used in the above process for producing a polyurethane resin is not particularly limited. For example, it may be one produced by using a compound having at least two active hydrogen groups (such as a polyhydric alcohol such as ethylene glycol, propylene glycol, glycerin, trimethylolpropane or pentaerythritol, an amine such as ethylenediamine, an alkanolamine such as ethylamine or diethanolamine, etc.) as a starting material and by an addition reaction of such a starting material with an alkylene oxide (such as ethylene oxide or propylene oxide) [e.g. Gunter Oertel, "Polyurethane Handbook" (1985) Hanser Publishers (Germany), method disclosed at p. 42-53].

The polyester polyol to be used in the above process for producing a polyurethane resin is not particularly limited. It may, for example, be one obtainable from a reaction of a dibasic acid with glycol, a waste from the production of nylon, a waste of trimethylolpropane or pentaerythritol, a waste of a phthalic acid-type polyester or a polyester polyol obtained by treating a waste product [e.g. Keiji Iwata "Polyurethane Resin Handbook" (1987) Nikkan Kogyo Shimbun, Ltd., disclosure at p. 117].

The polymer polyol to be used in the above process for producing a polyurethane resin is not particularly limited. It may, for example, be a polymer polyol obtained by reacting the above polyether polyol with an ethylenic unsaturated monomer (such as butadiene, acrylonitrile or styrene) in the presence of a radical polymerization catalyst.

The flame-resistant polyol to be used in the above process for producing a polyurethane resin is not particularly limited. It may, for example, be a phosphorus-containing polyol obtainable by adding an alkylene oxide to a phosphoric acid compound, a halogen-containing polyol obtainable by ring-opening polymerization of epichlorohydrin or trichlorobutylene oxide, or phenol polyol.

In the above process for producing a polyurethane resin, a polyol having an average hydroxy value within a range of from 20 to 1,000 mgKOH/g can be used, but for a flexible polyurethane resin or a semi-rigid polyurethane resin, one having an average hydroxy value within a range of from 20 to 100 mgKOH/g is preferably used, and for a rigid polyurethane resin, one having an average hydroxy value within a range of from 100 to 800 mgKOH/g is preferably used.

The polyisocyanate to be used in the above process for producing polyurethane resin may be conventional one and is not particularly limited. It may, for example, be an aromatic polyisocyanate such as toluene diisocyanate (hereinafter sometimes referred to as TDI), diphenylmethane diisocyanate (hereinafter sometimes referred to as MDI), naphthylene diisocyanate or a xylylene diisocyanate; an aliphatic polyisocyanate such as hexamethylene diisocyanate; an alicyclic polyisocyanate such as dicyclohexyl diisocyanate or isophorone diisocyanate; or a mixture thereof. Among them, preferred is TDI or its derivative, or MDI or its derivative, and they may be used alone or in combination as a mixture.

TDI or its derivative may, for example, be a mixture of 2,4-TDI and 2,6-TDI, or a terminal isocyanate prepolymer derivative of TDI. Whereas, MDI or its derivative may, for example, be a mixture of MDI and a polyphenylpolymethylene diisocyanate as its polymer, or a diphenylmethane diisocyanate derivative having a terminal isocyanate group.

Among the above isocyanates, for a flexible polyurethane resin or a semi-rigid polyurethane resin product, TDI or its derivative, and/or MDI or its derivative is preferably used. Whereas, for a rigid polyurethane resin, a mixture of MDI with a polyphenylpolymethylene diisocyanate as its polymer, is preferably used.

The mixing ratio of such a polyisocyanate to the polyol is not particularly limited, but when it is represented by an isocyanate index (i.e. [isocyanate groups]/[active hydrogen groups reactive with isocyanate groups]), it is usually preferably within a range of from 60 to 400, more preferably within a range of from 80 to 200.

In the above process for producing a polyurethane resin, as a catalyst, in addition to the catalyst composition of the present invention comprising the amine compound represented by the above formula (11) and the tertiary amine compound having a value of [blowing reaction rate constant/gelling reaction rate constant] of at least 0.5 not forming part of the claimed invention, other organic metal catalysts, carboxylic acid metal salt catalysts, tertiary amine catalysts, quaternary ammonium salt catalysts, etc. may be used in combination within a range not to depart from the concept of the present invention.

Such organic metal catalysts may be conventional ones and are not particularly limited. They may, for example, be stannous diacetate, stannous dioctoate, stannous dioleate, stannous dilaurate, dibutyltin oxide, dibutyltin diacetate, dibutyltin dilaurate, dibutyltin dichloride, dioctyltin dilaurate, lead octanoate, lead naphthenate, nickel naphthenate and cobalt naphthenate.

The above carboxylic acid metal salt catalysts may be conventional ones, and they may, for example, be alkali metal salts or alkaline earth metal salts of carboxylic acids. The carboxylic acids are not particularly limited, but they may, for example, be aliphatic mono- and di-carboxylic acids such as acetic acid, propionic acid, 2-ethylhexanoic acid and adipic acid; and aromatic mono- and di-carboxylic acids such as benzoic acid and phthalic acid. Further, metals to form carboxylic acid salts may, for example, be alkali metals such as lithium, sodium and potassium; or alkaline earth metals such as calcium and magnesium, as preferred examples.

The above tertiary amine catalysts not according to the claimed invention may be conventional ones, and they are

not particularly limited. They may, for example, be tertiary amine compounds such as N,N,N',N'-tetramethylethylenediamine, N,N,N',N'-tetramethylpropylenediamine, N,N,N',N",N"-pentamethyl-(3-aminopropyl)ethylenediamine, N,N,N',N",N"-pentamethyldipropylenetriamine, N,N,N',N'-tetramethylguanidine, 1,3,5-tris(N,N-dimethylaminopropyl) hexahydro-S-triazine, 1,8-diazabicyclo[5.4.0]undecene-7, triethylenediamine, N,N,N',N'-tetramethylhexamethylenediamine, N,N'-dimethylpiperazine, dimethylcyclohexylamine, N-methylmorpholine, N-ethylmorpholine, 1-methylimidazole, 1,2-dimethylimidazole, 1-isobutyl-2-methylimidazole and 1-dimethylaminopropylimidazole.

The above quaternary ammonium salt catalysts may be conventional ones and are not particularly limited. They may, for example, be a tetraalkylammonium halide such as tetramethylammonium chloride; a tetraalkylammonium hydroxide such as tetramethylammonium hydroxide; and a tetraalkylammonium organic salt such as tetramethylammonium 2-ethylhexanoate, 2-hydroxypropyltrimethylammonium formate, and 2-hydroxypropyltrimethylammonium 2-ethylhexanoate.

In the above process for producing a polyurethane resin, the catalyst composition of the present invention may be used alone or as mixed with the above-mentioned other catalysts. In the preparation of a catalyst mixture, a solvent such as dipropylene glycol, ethylene glycol, 1,4-butanediol or water may be used as the case requires. The amount of the solvent is not particularly limited, but it is preferably at most 3 times by weight to the total amount of the catalyst. If it exceeds 3 times by weight, it may present an adverse effect to the physical properties of the obtainable foam, and such is not desirable also from an economical reason. The catalyst composition thus prepared may be used as added to the polyol, or the individual components may separately be added to the polyol, and thus, the method for its use is not limited.

In the above process for producing a polyurethane resin, a blowing agent may be used as the case requires. Such a blowing agent is not particularly limited, but it may, for example, be a freon-type compound such as 1,1-dichloro-1-fluoroethane (HCFC-141b), 1,1,1,3,3-pentafluoropropane (HFC-245fa), 1,1,1,3,3-pentafluorobutane (HFC-365mfc), 1,1,2-tetrafluoroethane (HFC-134a), or 1,1,1,2,3,3,3-heptafluoropropane (HFC-227ea); a hydrofluoroether such as HFE-254pc; at least one member selected from the group consisting of a low boiling point hydrocarbon, water, liquefied carbon dioxide gas, dichloromethane, formic acid and acetone; or a mixture thereof.

As the low boiling point hydrocarbon, usually, a hydrocarbon having a boiling point of usually from -30 to 70°C is used, and its specific examples include propane, butane, pentane, cyclopentane, hexane and a mixture thereof.

The amount of the blowing agent is determined depending upon the desired density or physical properties of the foam. Specifically, it is selected so that the density of the obtainable foam becomes usually from 5 to 1,000 $kg/m^3$, preferably from 10 to 500 $kg/m^3$.

In the above process for producing a polyurethane resin, a surfactant may be used as a foam stabilizer, as the case requires. The surfactant to be used may, for example, be a conventional organic silicone type surfactant. Specifically, it may, for example, be a nonionic surfactant such as an organic siloxane-polyoxyalkylene copolymer or silicone-grease copolymer, or a mixture thereof. The amount of the surfactant is usually from 0.1 to 10 parts by weight, per 100 parts by weight of the polyol.

In the above process for producing a polyurethane resin, a crosslinking agent or chain extender may be used, as the case requires. The crosslinking agent or chain extender may, for example, be a low molecular weight polyhydric alcohol such as ethylene glycol, 1,4-butanediol or glycerin, a low molecular weight amine polyol such as diethanolamine or triethanolamine, or a polyamine such as ethylenediamine, xylylenediamine, methylenebisorthochloroaniline.

In the above process for producing a polyurethane resin, a flame retardant may be used as the case requires. The flame retardant to be used may, for example, be a reactive flame retardant like a phosphorus-containing polyol such as a propoxylated phosphoric acid obtained by an addition reaction of phosphoric acid with an alkylene oxide, or propoxylated dibutylpyrophosphoric acid; a tertiary phosphoric acid ester such as tricresyl phosphate; a halogen-containing tertiary phosphoric acid ester such as tris(2-chloroethyl) phosphate or tris(chloropropyl) phosphate; a halogen-containing organic compound such as dibromopropanol, dibromoneopentyl glycol or tetrabromo bisphenol A; or an inorganic compound such as antimony oxide, magnesium carbonate, calcium carbonate or aluminum phosphate. The amount of the flame retardant is not particularly limited and varies depending upon the desired flame retardancy, but it is usually from 4 to 20 parts by weight per 100 parts by weight of the polyol.

In the above process for producing a polyurethane resin, a colorant, an aging-preventive agent, or other conventional additives may be used, as the case requires. The types and amounts of these additives may be within usual ranges of such additives to be used.

In the above process for producing a polyurethane resin, a mixture solution having the above raw materials mixed is rapidly mixed and stirred and then injected into a proper container or mold, followed by blowing and molding. Mixing and stirring may be carried out by using a common stirring machine or a special polyurethane blowing machine. As the polyurethane blowing machine, a high pressure, low pressure or spray type machine can be used.

The polyurethane resin product may, for example, be an elastomer using no blowing agent, or a polyurethane foam using a blowing agent. The process for producing a polyurethane resin of the present invention is useful for the production of such a polyurethane foam product.

The polyurethane foam product may, for example, be a flexible polyurethane foam, a semi-rigid polyurethane

foam or a rigid polyurethane foam.

The process for producing a polyurethane resin of the present invention is particularly useful for the production of a car sheet made of a flexible polyurethane foam to be used as an interior material for an automobile, an instrument panel or handle made of a semi-rigid polyurethane foam, or a heat insulating material made of a rigid polyurethane foam.

Here, in the present invention, the flexible polyurethane foam usually means a highly air permeable reversibly deformable foam having an open cell structure [Gunter Oertel, "Polyurethane Handbook" (1985 edition) Hanser Publishers (Germany), p. 161-233, and Keiji Iwata "Polyurethane Resin Handbook" (1987 first edition) Nikkan Kogyo Shimbun, Ltd., p. 150-221].

The physical properties of the flexible urethane foam are not particularly limited, but usually the density is within a range of from 10 to 100 $kg/m^3$, the compression strength (ILD25%) is within a range of from 200 to 8,000 kPa, and the elongation is within a range of from 80 to 500%. Here, ILD (Indentation Load Deflection) 25% is measured by a resistance at the time when a harder material (e.g. a metal disk with a radius of 10 cm) is pushed in against a urethane foam as a sample, by 25% of the sample thickness.

Whereas, the semi-rigid polyurethane foam means a highly air permeable reversibly deformable foam having an open cell structure like the flexible polyurethane foam, although the foam density and compression strength are higher than the flexible polyurethane foam [Gunter Oertel, "Polyurethane Handbook" (1985 edition) Hanser Publishers (Germany), p. 223-233, and Keiji Iwata "Polyurethane Resin Handbook" (1987 first edition) Nikkan Kogyo Shimbun, Ltd., p. 211-221].

Further, the polyol and isocyanate materials to be used are also the same as for a flexible polyurethane foam, and accordingly, the semi-rigid polyurethane foam is usually classified in a soft polyurethane foam.

The physical properties of the semi-rigid urethane foam are not particularly limited, but usually, the density is within a range of from 40 to 800 $kg/m^3$, the compression strength (ILD25%) is within a range of from 10 to 200 kPa, and the elongation is within a range of from 40 to 200%. In the present invention, a flexible polyurethane foam may sometimes contain a semi-rigid polyurethane foam from the raw materials to be used and physical properties of the foam.

Further, the rigid polyurethane foam means a reversibly deformable foam having a highly crosslinked closed cell structure [Gunter Oertel, "Polyurethane Handbook" (1985 edition) Hanser Publishers (Germany), p. 234-313, and Keiji Iwata "Polyurethane Resin Handbook" (1987 first edition) Nikkan Kogyo Shimbun, Ltd., p. 224-283].

The physical properties of the rigid urethane foam are not particularly limited, but usually, the density is within a range of from 10 to 100 $kg/m^3$, and the compression strength is within a range of from 50 to 1,000 kPa.

## EXAMPLES

Firstly, the process for producing a hydroxyalkyltriethylenediamine or hydroxytriethylenediamine not forming part of the present invention as well as the process for producing a hydroxyalkylpiperazine and/or hydroxypiperazine not forming part of the present invention, will be described in further detail with reference to the following Examples.

### PREPARATION EXAMPLE 1 (Preparation of dihydroxypropylpiperazine)

Into a 200 ml three-necked flask, 86.1 g (1.0 mol) of piperazine and 100 ml of methanol as a solvent were charged, and in a nitrogen atmosphere, 22.2 g (0.3 mol) of glycidol was dropwise added over a period of 4 hours. The three-necked flask was held in an oil bath, and the temperature of the reaction solution was maintained at 60°C. After completion of the dropwise addition of glycidol, methanol as the solvent and unreacted piperazine in the reaction solution were distilled off by simple distillation. The product was vacuum-dried to obtain 45.2 g of a white viscous solid. This substance was confirmed to be dihydroxypropylpiperazine represented by the above formula (3a) (hereinafter referred to as DHPP-3a) by the gas chromatography mass analysis and the nuclear magnetic resonance analysis.

### PREPARATION EXAMPLE 2 (Preparation of dihydroxypropylpiperazine)

86.1 g (1.0 mol) of piperazine, 92.1 g (1.0 mol) of glycerin, 5.0 g of aluminum phosphate (manufactured by Wako Pure Chemical Industries, Ltd., for chemical application) as a catalyst and 600 ml of water as a solvent were charged into a 1,000 ml autoclave and heated to 280°C in a nitrogen atmosphere. At that time, the reactor pressure was 6.0 MPa (gauge pressure, the same applies hereinafter). The reaction time was 2 hours. After completion of the reaction, water as a solvent, unreacted piperazine, glycerin and by-products in the reaction solution were distilled off by distillation to obtain a desired product (white viscous solid: 16.4 g). This substance was confirmed to be DHPP-3a by the gas chromatography mass analysis and the nuclear magnetic resonance analysis.

PREPARATION EXAMPLE 3 (Preparation of dihydroxypropylpiperazine)

86.1 g (1.0 mol) of piperazine, 55.3 g (0.5 mol) of chloropropanediol and 200 ml of methanol as a solvent were charged into a 500 ml three-necked flask and heated to 60°C in a nitrogen atmosphere. At that time, the reactor pressure was the atmospheric pressure. The reaction time was 16 hours. After completion of the reaction, a sodium hydroxide aqueous solution having a concentration of 5 mol/L (100 ml) was added for phase separation of the reaction solution, whereupon the product contained in the organic layer was extracted with 1-butanol. Water as a solvent, unreacted piperazine and by-products in the reaction solution were distilled off by distillation to obtain the desired product (white viscous solid: 56.1 g). This substance was confirmed to be DHPP-3a by the gas chromatography mass analysis and the nuclear magnetic resonance analysis.

PREPARATION EXAMPLE 4 (Preparation of dihydroxypropylpiperazine)

86.1 g (1.0 mol) of piperazine, 90.1 g (1.0 mol) of dihydroxyacetone, 10 g (dries weight: 5.0 g) of Raney nickel as a catalyst and 100 ml of ethanol as a solvent were charged into a 1,000 ml autoclave and heated to 90°C in a nitrogen atmosphere. At that time, the reactor pressure was 11.0 MPa. The reaction time was 3 hours. After completion of the reaction, ethanol as a solvent, unreacted piperazine, etc. in the reaction solution were distilled off by simple distillation to obtain 105.7 g of a brown transparent liquid. This substance was confirmed to be dihydroxypropylpiperazine represented by the above formula (3b) (hereinafter referred to as DHPP-3b) by the gas chromatography mass analysis and the nuclear magnetic resonance analysis.

PREPARATION EXAMPLE 5 (Preparation of dihydroxypropylpiperazine)

Into a 2,000 ml three-necked flask, 86.1 g (1.0 mol) of piperazine, 119.5 g (0.5 mol) of diethyl bromomalonate and 800 ml of acetonitrile as a solvent were charged and heated to 80°C for reaction. The reactor pressure was the atmospheric pressure, and the reaction time was 24 hours. The reaction solution was subjected to filtration, and the solvent was distilled off by an evaporator, followed by purification by means of a silica gel column chromatography to obtain 85.5 g of a slightly yellow transparent intermediate product. This substance was confirmed to be a monoalkylester of piperazine (i.e. diethyl 2-(piperazin-1-yl)malonate by the nuclear magnetic resonance analysis. 85.5 g (0.35 mol) of this intermediate product was reduced by means of lithium aluminum hydride (0.70 mol) in a dehydrated tetrahydrofuran solvent. Then, the reaction solution was subjected to filtration, and the solvent was distilled off by an evaporator. Then, the residue was vacuum-dried to obtain 39.5 g of a brown transparent liquid. This substance was confirmed to be DHPP-3b by the gas chromatography mass analysis and the nuclear magnetic resonance analysis. EXAMPLE 1 (not forming part of the invention)

16.0 g (0.10 mol) of DHPP-3a obtained in Preparation Example 1, 100 ml of water as a solvent and 5.0 g of aluminum phosphate (manufactured by Wako Pure Chemical Industries, Ltd., for chemical application) as a catalyst were charged into a 200 ml autoclave and heated to 280°C in a nitrogen atmosphere. At that time, the reactor pressure was 8.0 MPa. The reaction time was 2 hours.

The reaction product was analyzed by gas chromatography. As a result, the conversion of DHPP-3a was 59%, and the selectivity for products was such that hydroxymethyltriethylenediamine was 85%, and triethylenediamine formed by detachment of a hydroxymethyl group was 13%.

EXAMPLE 2 (not forming part of the invention)

A reaction was carried out in the same manner as in Example 1 except that instead of DHPP-3a obtained in Preparation Example 1, DHPP-3a obtained in Preparation Example 2 was used.

The reaction product was analyzed by gas chromatography. As a result, the conversion of DHPP-3a was 60%, and the selectivity for products was such that hydroxymethyltriethylenediamine was 84%, and triethylenediamine formed by detachment of a hydroxymethyl group was 14%.

EXAMPLE 3 (not forming part of the invention)

A reaction was carried out in the same manner as in Example 1 except that instead of DHPP-3a obtained in Preparation Example 1, DHPP-3a obtained in Preparation Example 3 was used.

The reaction product was analyzed by gas chromatography. As a result, the conversion of DHPP-3a was 61%, and the selectivity for products was such that hydroxymethyltriethylenediamine was 85%, and triethylenediamine formed by detachment of a hydroxymethyl group was 14%.

EXAMPLE 4 (not forming part of the invention)

16.0 g (0.10 mol) of DHPP-3a obtained in Preparation Example 1, 100 ml of water as a solvent and 5.0 g of phenyl phosphonic acid (manufactured by Wako Pure Chemical Industries, Ltd., for chemical application) as a catalyst were charged into a 200 ml autoclave and heated to 280°C in a nitrogen atmosphere. At that time, the reactor pressure was 8.0 MPa. The reaction time was 2 hours.

The reaction product was analyzed by gas chromatography. As a result, the conversion of DHPP-3a was 67%, and the selectivity for products was such that hydroxymethyltriethylenediamine was 76%, and triethylenediamine formed by detachment of a hydroxymethyl group was 24%.

COMPARATIVE EXAMPLE 1

16.0 g (0.10 mol) of DHPP-3a obtained in Preparation Example 1 and 100 ml of water as a solvent were charged into a 200 ml autoclave without adding any catalyst and, after nitrogen purging, heated to 280°C. At that time, the reactor pressure was 8.0 MPa. The reaction time was 2 hours.

The reaction product was analyzed by gas chromatography, whereby the conversion of DHPP-3a was found to be 0%.

COMPARATIVE EXAMPLE 2

16.0 g (0.10 mol) of DHPP-3a obtained in Preparation Example 1, 100 ml of water as a solvent and 10.0 g (dried weight: 5.0 g) of a Raney nickel catalyst were charged into a 200 ml autoclave and, after nitrogen purging, heated to 150°C under hydrogen pressure. At that time, the reactor pressure was 10.0 MPa. The reaction time was 2 hours.

The reaction product was analyzed by gas chromatography, whereby the conversion of DHPP-3a was found to be 0%.

EXAMPLE 5 (not forming part of the invention)

16.0 g (0.10 mol) of DHPP-3b obtained in Preparation Example 4, 100 ml of water as a solvent and 5.0 g of aluminum phosphate (manufactured by Wako Pure Chemical Industries, Ltd., for chemical application) as a catalyst were charged into a 200 ml autoclave and heated to 280°C in a nitrogen atmosphere. At that time, the reactor pressure was 8.0 MPa. The reaction time was 2 hours.

The reaction product was analyzed by gas chromatography. As a result, the conversion of DHPP-3b was 84%, and the selectivity for products was such that hydroxymethyltriethylenediamine was 90%, and triethylenediamine formed by detachment of a hydroxymethyl group was 5%.

EXAMPLE 6 (not forming part of the invention)

16.0 g (0.10 mol) of DHPP-3b obtained in Preparation Example 5, 100 ml of water as a solvent and 5.0 g of aluminum phosphate (manufactured by Wako Pure Chemical Industries, Ltd., for chemical application) as a catalyst were charged into a 200 ml autoclave and heated to 280°C in a nitrogen atmosphere. At that time, the reactor pressure was 8.0 MPa. The reaction time was 2 hours.

The reaction product was analyzed by gas chromatography. As a result, the conversion of DHPP-3b was 85%, and the selectivity for products was such that hydroxymethyltriethylenediamine was 91%, and triethylenediamine formed by detachment of a hydroxymethyl group was 4%.

EXAMPLE 7 (not forming part of the invention)

16.0 g (0.10 mol) of DHPP-3b obtained in Preparation Example 4, 100 ml of water as a solvent and 5.0 g of phenyl phosphonic acid (manufactured by Wako Pure Chemical Industries, Ltd., for chemical application) as a catalyst were charged into a 200 ml autoclave and heated to 280°C in a nitrogen atmosphere. At that time, the reactor pressure was 8.0 MPa. The reaction time was 2 hours.

The reaction product was analyzed by gas chromatography. As a result, the conversion of DHPP-3b was 75%, and the selectivity for products was such that hydroxymethyltriethylenediamine was 73%, and triethylenediamine formed by detachment of a hydroxymethyl group was 21%.

COMPARATIVE EXAMPLE 3

16.0 g (0.10 mol) of DHPP-3b obtained in Preparation Example 4 and 100 ml of water as a solvent were charged into a 200 ml autoclave without adding any catalyst and, after nitrogen purging, heated to 280°C. At that time, the reactor pressure was 8.0 MPa. The reaction time was 2 hours.
The reaction product was analyzed by gas chromatography, whereby the conversion of DHPP-3b was found to be 0%.

COMPARATIVE EXAMPLE 4

16.0 g (0.10 mol) of DHPP-3b obtained in Preparation Example 4, 100 ml of water as a solvent and 10.0 g (dried weigh: 5.0 g) of a Raney nickel catalyst were charged into a 200 ml autoclave and after nitrogen purging, heated to 150°C under hydrogen pressure. At that time, the reactor pressure was 10.0 MPa. The reaction time was 2 hours.
The reaction product was analyzed by gas chromatography, whereby the conversion of DHPP-3b was found to be 0%.

PREPARATION EXAMPLE 6 (Preparation of dihydroxypropylethylenediamine)

Into a 200 ml three-necked flask, 120.2 g (2.0 mol) of ethylenediamine and 100 ml of methanol as a solvent were charged, and 44.4 g (0.6 mol) of glycidol was dropwise added over a period of 4 hours in a nitrogen atmosphere. The three-necked flask was held in an oil bath, and the temperature of the reaction solution was maintained at 60°C. After completion of the dropwise addition of glycidol, methanol as a solvent and unreacted ethylenediamine in the reaction solution were distilled off by simple distillation. Further, the product was vacuum-dried to obtain 72.2 g of a yellowish white solid. This substance was confirmed to be 2,3-dihydroxypropylethylenediamine represented by the above formula (9a) (hereinafter referred to as 2,3-DHPEDA) by the gas chromatography mass analysis and the nuclear magnetic resonance analysis.

PREPARATION EXAMPLE 7 (Preparation of dihydroxypropylethylenediamine)

480.8 g (8.0 mol) of ethylenediamine, 90.1 g (1.0 mol) of dihydroxyacetone, 30 g (dried weight: 15.0 g) of Raney nickel as a catalyst and 200 ml of ethanol as a solvent were charged into a 1,000 ml autoclave and heated to 90°C in a hydrogen atmosphere. At that time, the reactor pressure was 11.0 MPa. The reaction time was 3 hours. After completion of the reaction, ethanol as a solvent, unreacted ethylenediamine, etc. in the reaction solution were distilled off by simple distillation to obtain 80.7 g of a yellowish white solid. This substance was confirmed to be 1,3-dihydroxypropylethylenediamine represented by the above formula (9b) (hereinafter referred to as 1,3-DHPEDA) by the gas chromatography mass analysis and the nuclear magnetic resonance analysis.

EXAMPLE 8 (not forming part of the invention)

13.4 g (0.10 mol) of 2,3-DHPEDA obtained in Preparation Example 6, 100 ml of water as a solvent and 5.0 g of aluminum phosphate (manufactured by Wako Pure Chemical Industries, Ltd., for chemical application) as a catalyst were charged into a 200 ml autoclave and heated to 280°C in a nitrogen atmosphere. At that time, the reactor pressure was 6.0 MPa. The reaction time was 2 hours.
The reaction product was analyzed by gas chromatography. As a result, the conversion of 2,3-DHPEDA was 80%, and the selectivity for products was such that hydroxymethylpiperazine was 70%, piperazine formed by detachment of a hydroxymethyl group was 12%, and ethylenediamine was 15%.

EXAMPLE 9 (not forming part of the invention)

13.4 g (0.10 mol) of 2,3-DHPEDA obtained in Preparation Example 6, 100 ml of water as a solvent and 5.0 g of phenyl phosphonic acid (manufactured by Wako Pure Chemical Industries, Ltd., for chemical application) as a catalyst were charged into a 200 ml autoclave and heated to 280°C in a nitrogen atmosphere. At that time, the reactor pressure was 8.0 MPa. The reaction time was 2 hours.
The reaction product was analyzed by gas chromatography. As a result, the conversion of DHPEDA was 65%, and the selectivity for products was such that hydroxymethylpiperazine was 56%, piperazine formed by detachment of a hydroxymethyl group was 14%, and ethylenediamine was 27%.

EXAMPLE 10 (not forming part of the invention)

13.4 g (0.10 mol) of 1,3-DHPEDA obtained in Preparation Example 7, 100 ml of water as a solvent and 5.0 g of aluminum phosphate (manufactured by Wako Pure Chemical Industries, Ltd., for chemical application) as a catalyst were charged into a 200 ml autoclave and heated to 280°C in a nitrogen atmosphere. At that time, the reactor pressure was 6.0 MPa. The reaction time was 2 hours.

The reaction product was analyzed by gas chromatography. As a result, the conversion of 1,3-DHPEDA was 80%, and the selectivity for products was such that hydroxymethylpiperazine was 70%, piperazine formed by detachment of a hydroxymethyl group was 12%, and ethylenediamine was 15%.

EXAMPLE 11 (not forming part of the invention)

At a center portion of a quartz glass tube having an inner diameter of 60 mm, 20 ml of the same aluminum phosphate as one used in Example 8 (manufactured by Wako Pure Chemical Industries, Ltd., for chemical application) was filled, and above and below it, raschig ring packing with an outer diameter of 3 mm was packed. While the temperature of the aluminum phosphate layer and the raschig ring layers was kept at 300°C, from the top, an aqueous solution of 2,3-dihydroxypropylethylenediamine (2,3-DHPEDA) obtained in Preparation Example 6 [dihydroxypropylethylenediamine:water=13:87 by weight ratio] was dropwise added at a rate of GHSV=1,500 $h^{-1}$ (GHSV means gas hourly space velocity). The obtained reaction solution was analyzed by gas chromatography. As a result, the conversion of 2,3-DHPEDA was 85%, and the selectivity for products was such that hydroxymethylpiperazine was 76%, piperazine formed by detachment of a hydroxymethyl group was 9%, and ethylenediamine was 12%.

COMPARATIVE EXAMPLE 5

13.4 g (0.10 mol) of 2,3-DHPEDA obtained in Preparation Example 6 and 100 ml of water as a solvent were charged into a 200 ml autoclave without adding any catalyst and, after nitrogen purging, heated to 280°C. At that time, the reactor pressure was 6.0 MPa. The reaction time was 2 hours.

The reaction product was analyzed by gas chromatography, whereby the conversion of 2,3-DHPEDA was found to be 0%.

COMPARATIVE EXAMPLE 6

13.4 g (0.10 mol) of 1,3-DHPEDA obtained in Preparation Example 7 and 100 ml of water as a solvent were charged into a 200 ml autoclave without adding any catalyst and, after nitrogen purging, heated to 280°C. At that time, the reactor pressure was 6.0 MPa. The reaction time was 2 hours.

The reaction product was analyzed by gas chromatography, whereby the conversion of 1,3-DHPEDA was found to be 0%.

EXAMPLE 12 (not forming part of the invention)

13.4 g (0.10 mol) of 1,3-DHPEDA obtained in Preparation Example 7, 100 ml of ethanol as a solvent and 10.0 g (dried weight: 5.0 g) of a Raney nickel catalyst were charged into a 200 ml autoclave and, after nitrogen purging, heated to 150°C under hydrogen pressure. At that time, the reactor pressure was 15.0 MPa. The reaction time was 3 hours.

The reaction product was analyzed by gas chromatography. As a result, the conversion of 1,3-DHPEDA was 24%, and the selectivity for products was such that hydroxymethylpiperazine was 40%, piperazine formed by detachment of a hydroxymethyl group was 23%, and ethylenediamine was 37%.

PREPARATION EXAMPLE 8 (Preparation of dihydroxypropylethylenediamine)

Into a 10 L autoclave, 1,202 g (20 mol) of ethylenediamine and 1,000 ml of methanol as a solvent were charged, and 663 g (6 mol) of chloropropanediol was dropwise added over a period of 2 hours in a nitrogen atmosphere. The autoclave was heated, and the temperature of the reaction solution was adjusted to 100°C. At that time, the reactor pressure was 0.5 MPa. After completion of the dropwise addition of chloropropanediol, the aging time was 4 hours. This reaction solution was neutralized with a 48% sodium hydroxide aqueous solution (333 ml), and then, a filtration operation was carried out. A low boiling fraction of the filtrate obtained by this operation was distilled off by an evaporator, followed by distillation for purification to obtain 833 g of a slightly yellow solid. This substance was confirmed to be 2,3-dihydroxypropylethylenediamine represented by the above formula (9a) (hereinafter referred to as 2,3-DHPEDA) by the gas chromatography mass analysis and the nuclear magnetic resonance analysis.

PREPARATION EXAMPLE 9 (Preparation of dihydroxypropylethylenediamine)

Into a 10 L autoclave, 1,202 g (20 mol) of ethylenediamine and 1,000 ml of methanol as a solvent were charged, and 444 g (6 mol) of glycidol was dropwise added over a period of 4 hours in a nitrogen atmosphere. The autoclave was heated, and the temperature of the reaction solution was maintained at 60°C. After completion of the dropwise addition of glycidol, methanol as a solvent and unreacted ethylenediamine in the reaction solution were distilled off by simple distillation. The product was vacuum-dried to obtain 722 g of a yellowish white solid. This substance was confirmed to be 2,3-DHPEDA represented by the above formula (9a) by the gas chromatography mass analysis and the nuclear magnetic resonance analysis. EXAMPLE 13 (not forming part of the invention)

124 g (0.92 mol) of 2,3-DHPEDA obtained in Preparation Example 8, 500 ml of water as a solvent and 6.2 g of Raney copper (manufactured by Kawaken Fine Chemicals Co., Ltd., tradename: CDT-60) as a catalyst were charged into a 1,000 ml autoclave and heated to 165°C in a hydrogen atmosphere. At that time, the reactor pressure was 3.5 MPa. The reaction time was 4 hours.

The reaction product was analyzed by gas chromatography. As a result, the conversion of 2,3-DHPEDA was 96.2%, and the selectivity for a product was such that hydroxymethylpiperazine was 68%.

EXAMPLE 14 (not forming part of the invention)

124 g (0.92 mol) of 2,3-DHPEDA obtained in Preparation Example 9, 500 ml of water as a solvent and 6.2 g of Raney copper (manufactured by Kawaken Fine Chemicals Co., Ltd., tradename: CDT-60) as a catalyst were charged into a 1,000 ml autoclave and heated to 165°C in a hydrogen atmosphere. At that time, the reactor pressure was 3.5 MPa. The reaction time was 4 hours.

The reaction product was analyzed by gas chromatography. As a result, the conversion of 2,3-DHPEDA was 95.9%, and the selectivity for a product was such that hydroxymethylpiperazine was 67%.

EXAMPLE 15 (not forming part of the invention)

60 g (0.45 mol) of 2,3-DHPEDA obtained in Preparation Example 8, 540 ml of water as a solvent and 6.0 g of Raney copper (manufactured by Kawaken Fine Chemicals Co., Ltd., tradename: CDT-60) as a catalyst were charged into a 1,000 ml autoclave and heated to 165°C in a hydrogen atmosphere. At that time, the reactor pressure was 3.5 MPa. The reaction time was 4 hours.

The reaction product was analyzed by gas chromatography. As a result, the conversion of 2,3-DHPEDA was 99.7%, and the selectivity for a product was such that hydroxymethylpiperazine was 70%.

EXAMPLE 16 (not forming part of the invention)

180 g (1.50 mol) of 2,3-DHPEDA obtained in Preparation Example 8, 420 ml of water as a solvent and 7.2 g of Raney copper (manufactured by Kawaken Fine Chemicals Co., Ltd., tradename: CDT-60) as a catalyst were charged into a 1,000 ml autoclave and heated to 165°C in a hydrogen atmosphere. At that time, the reactor pressure was 3.5 MPa. The reaction time was 4 hours.

The reaction product was analyzed by gas chromatography. As a result, the conversion of 2,3-DHPEDA was 84.8%, and the selectivity for a product was such that hydroxymethylpiperazine was 52%.

EXAMPLE 17 (not forming part of the invention)

200 ml of water as a solvent and 20.0 g of Raney copper (manufactured by Kawaken Fine Chemicals Co., Ltd., tradename: CDT-60) as a catalyst were charged into a 1,000 ml autoclave and heated to 165°C in a hydrogen atmosphere. At that time, the reactor pressure was 3.5 MPa.

Then, 200 g (1.50 mol) of 2,3-DHPEDA obtained in Preparation Example 8 as dissolved in 267 ml of water was dropwise supplied into the autoclave by a metering pump. The time for the dropwise addition was 4 hours.

The reaction product was analyzed by gas chromatography. As a result, the conversion of 2,3-DHPEDA was 100%, and the selectivity for a product was such that hydroxymethylpiperazine was 61%.

EXAMPLE 18 (not forming part of the invention)

50 g (0.37 mol) of 2,3-DHPEDA obtained in Preparation Example 8, 50 ml of water as a solvent and 2.5 g of Raney nickel (manufactured by Evonik Degussa Japan, tradename: B111W) as a catalyst were charged into a 200 ml autoclave and heated to 165°C in a hydrogen atmosphere. At that time, the reactor pressure was 3.5 MPa. The reaction time was 4

hours.

The reaction product was analyzed by gas chromatography. As a result, the conversion of 2,3-DHPEDA was 53.1%, and the selectivity for a product was such that hydroxymethylpiperazine was 21%.

COMPARATIVE EXAMPLE 7

201 g (1.50 mol) of 2,3-DHPEDA obtained in Preparation Example 8, 201 ml of water as a solvent and 10.1 g of copper chromium catalyst (manufactured by JGC C&C, tradename: N203S) as a catalyst were charged into a 1,000 ml autoclave and heated to 165°C in a hydrogen atmosphere. At that time, the reactor pressure was 3.5 MPa. The reaction time was 4 hours.

The reaction product was analyzed by gas chromatography. As a result, the conversion of 2,3-DHPEDA was so low that it could not be separated from by-products. The yield of hydroxymethylpiperazine was 5.6%.

COMPARATIVE EXAMPLE 8

200 g (1.49 mol) of 2,3-DHPEDA obtained in Preparation Example 8, 200 ml of water as a solvent and 10.0 g of copper chromium catalyst (manufactured by JGC C&C, tradename: N203S) as a catalyst were charged into a 1,000 ml autoclave and heated to 200°C in a hydrogen atmosphere. At that time, the reactor pressure was 3.5 MPa. The reaction time was 4 hours.

The reaction product was analyzed by gas chromatography. As a result, the conversion of 2,3-DHPEDA was so low that it could not be separated from by-products. The yield of hydroxymethylpiperazine was 5.3%.

EXAMPLE 19 (not forming part of the invention)

(1) Preparation of 1-hydroxyethyl-3-hydroxymethylpiperazine

116.2 g (1.0 mol) of hydroxymethylpiperazine prepared by the method disclosed in J. Med. Chem. 36, 2075 (1993) and 200 ml of methanol as a solvent were charged into a 1,000 ml autoclave, and 44.1 g (1.0 mol) of ethylene oxide was dropwise added in a nitrogen atmosphere. Here, the autoclave was held in an ice water bath to adjust the reaction temperature at the time of initiation of the dropwise addition to be 0°C. At that time, the reactor pressure was 0.1 MPa. The reaction time was 3 hours. After completion of the reaction, the autoclave was heated and aged at 60°C for 3 hours. Then, ethanol as a solvent, unreacted 2-hydroxymethylpiperazine, etc. in the reaction solution were distilled off by simple distillation. The product was vacuum-dried to obtain 154.6 g of a white solid. This substance was confirmed to be 1-hydroxyethyl-3-hydroxymethylpiperazine (hereinafter referred to as HEHMP) corresponding to a dihydroxyalkylpiperazine derivative represented by the above formula (4), by the gas chromatography mass analysis and the nuclear magnetic resonance analysis.

(2) Preparation of 2-hydroxymethyltriethylenediamine

16.0 g (0.10 mol) of the above HEHMP, 100 ml of water as a solvent and 5.0 g of aluminum phosphate (manufactured by Wako Pure Chemical Industries, Ltd., for chemical application) as a catalyst were charged into a 200 ml autoclave and heated to 280°C in a nitrogen atmosphere. At that time, the reactor pressure was 8.0 MPa. The reaction time was 2 hours.

The reaction product was analyzed by gas chromatography. As a result, the conversion of HEHMP was 98%, and the selectivity for products was such that 2-hydroxymethyltriethylenediamine was 92%, and triethylenediamine formed by detachment of a hydroxymethyl group was not more than 1%.

EXAMPLE 20 (not forming part of the invention)

16.0 g (0.10 mol) of the above HEHMP obtained in Example 19(1), 100 ml of water as a solvent and 5.0 g of phenylphosphonic acid (manufactured by Wako Pure Chemical Industries, Ltd., for chemical application) as a catalyst were charged into a 200 ml autoclave and heated to 280°C in a nitrogen atmosphere. At that time, the reactor pressure was 8.0 MPa. The reaction time was 2 hours.

The reaction product was analyzed by gas chromatography. As a result, the conversion of HEHMP was 88%, and the selectivity for products was such that hydroxymethyltriethylenediamine was 85%, and triethylenediamine formed by detachment of a hydroxymethyl group was 5%.

COMPARATIVE EXAMPLE 9

16.0 g (0.10 mol) of HEHMP obtained in Example 19(1) and 100 ml of water as a solvent were charged into a 200 ml autoclave without adding any catalyst and, after nitrogen purging, heated to 280°C. At that time, the reactor pressure was 8.0 MPa. The reaction time was 2 hours.
The reaction product was analyzed by gas chromatography, whereby the conversion of HEHMP was found to be 0%.

COMPARATIVE EXAMPLE 10

16.0 g (0.10 mol) of HEHMP obtained in Example 19(1), 100 ml of water as a solvent and 10.0 g (dried weight: 5.0 g) of a Raney nickel catalyst were charged into a 200 ml autoclave and, after nitrogen purging, heated to 150°C under hydrogen pressure. At that time, the reactor pressure was 10.0 MPa. The reaction time was 2 hours.
The reaction product was analyzed by gas chromatography, whereby the conversion of HEHMP was found to be 0%.
Now, the second process for producing a hydroxyalkyltriethylenediamine of the present invention will be described in further detail with reference to the following Examples, but it should be understood that the present invention is by no means thereby restricted.

EXAMPLE 21 (not forming part of the invention)

15.5 g (0.18 mol) of piperazine, 16.6 g (0.18 mol) of glycerin, 135 mol of water as a solvent and 5.0 g of phenyl phosphonic acid (manufactured by Wako Pure Chemical Industries, Ltd., for chemical application) as a catalyst were charged into a 200 ml autoclave and heated to 280°C in a nitrogen atmosphere. At that time, the reactor pressure was 6.0 MPa. The reaction time was 12 hours.
The reaction product was analyzed by gas chromatography, whereby the conversion of piperazine was found to be 41%, and the yield of hydroxymethyltriethylenediamine was 10%.

EXAMPLE 22 (not forming part of the invention)

15.5 g (0.18 mol) of piperazine, 16.6 g (0.18 mol) of glycerin, 135 ml of water as a solvent and 5.0 g of aluminum phosphate (manufactured by Wako Pure Chemical Industries, Ltd., for chemical application) as a catalyst were charged into a 200 ml autoclave and heated to 280°C in a nitrogen atmosphere. At that time, the reactor pressure was 6.0 MPa. The reaction time was 12 hours.
The reaction product was analyzed by gas chromatography, whereby the conversion of piperazine was found to be 53%, and the yield of hydroxymethyltriethylenediamine was 12%.

EXAMPLE 23 (not forming part of the invention)

15.5 g (0.18 mol) of piperazine, 16.6 g (0.18 mol) of glycerin, 135 ml of water as a solvent and 5.0 g of silica-alumina (manufactured by JGC C&C, for chemical application) as a catalyst were charged into a 200 ml autoclave and heated to 280°C in a nitrogen atmosphere. At that time, the reactor pressure was 6.0 MPa. The reaction time was 12 hours.
The reaction product was analyzed by gas chromatography, whereby the conversion of piperazine was found to be 38%, and the yield of hydroxymethyltriethylenediamine was 10%.

EXAMPLE 24 (not forming part of the invention)

15.5 g (0.18 mol) of piperazine, 82.9 g (0.90 mol) of glycerin, 135 ml of water as a solvent and 5.0 g of aluminum phosphate (manufactured by Wako Pure Chemical Industries, Ltd., for chemical application) as a catalyst were charged into a 200 ml autoclave and heated to 280°C in a nitrogen atmosphere. At that time, the reactor pressure was 6.0 MPa. The reaction time was 12 hours.
The reaction product was analyzed by gas chromatography, whereby the conversion of piperazine was found to be 89%, and the yield of hydroxymethyltriethylenediamine was 8%.

EXAMPLE 25 (not forming part of the invention)

77.5 g (0.90 mol) of piperazine, 16.6 g (0.18 mol) of glycerin, 135 ml of water as a solvent and 5.0 g of aluminum phosphate (manufactured by Wako Pure Chemical Industries, Ltd., for chemical application) as a catalyst were charged

into a 200 ml autoclave and heated to 280°C in a nitrogen atmosphere. At that time, the reactor pressure was 6.0 MPa. The reaction time was 12 hours.

The reaction product was analyzed by gas chromatography, whereby the conversion of piperazine was found to be 11%, and the selectivity was such that hydroxymethyltriethylenediamine was 2%.

COMPARATIVE EXAMPLE 11

15.5 g (0.18 mol) of piperazine, 16.6 g (0.18 mol) of glycerin and 135 ml of water as a solvent were charged into a 200 ml autoclave without adding any catalyst and heated to 280°C in a nitrogen atmosphere. At that time, the reactor pressure was 6.0 MPa. The reaction time was 12 hours.

The reaction product was analyzed by gas chromatography, whereby the conversion of piperazine was found to be 0%.

COMPARATIVE EXAMPLE 12

15.5 g (0.18 mol) of piperazine, 16.6 g (0.18 mol) of glycerin, 135 ml of water as a solvent and 12.5 g of Raney nickel (manufactured by Degussa, B111W) as a catalyst were charged into a 200 ml autoclave and heated to 280°C in a nitrogen atmosphere. At that time, the reactor pressure was 6.0 MPa. The reaction time was 12 hours.

The reaction product was analyzed by gas chromatography, whereby the conversion of piperazine was found to be 0%.

COMPARATIVE EXAMPLE 13

15.5 g (0.18 mol) of piperazine, 16.6 g (0.18 mol) of glycerin, 135 ml of water as a solvent and 5.0 g of titanium(IV) oxide (manufactured by Wako Pure Chemical Industries, Ltd., for chemical application) as a catalyst were charged into a 200 ml autoclave and heated to 280°C in a nitrogen atmosphere. At that time, the reactor pressure was 6.0 MPa. The reaction time was 12 hours.

The reaction product was analyzed by gas chromatography, whereby the conversion of piperazine was found to be 0%.

COMPARATIVE EXAMPLE 14

15.5 g (0.18 mol) of piperazine, 16.6 g (0.18 mol) of glycerin, 135 ml of water as a solvent and 5.0 g of copper(II) oxide (manufactured by Wako Pure Chemical Industries, Ltd., for chemical application) as a catalyst were charged into a 200 ml autoclave and heated to 280°C in a nitrogen atmosphere. At that time, the reactor pressure was 6.0 MPa. The reaction time was 12 hours.

The reaction product was analyzed by gas chromatography, whereby the conversion of piperazine was found to be 0%.

As is evident from the above Comparative Examples 11 to 14, when the acid catalyst of the present invention was not used, hydroxymethyltriethylenediamine was not obtained.

COMPARATIVE EXAMPLE 15

In accordance with method disclosed in Patent Document 1, preparation of hydroxymethyltriethylenediamine was carried out. Into a 2 L separable flask, 43.1 g (0.5 mol) of piperazine and 151.8 g (1.5 mol) of triethylamine were charged and diluted with toluene (1,000 ml). After nitrogen purging, 130.0 g of ethyl 2,3-dibromopropionate (manufactured by Tokyo Chemical Industry Co., Ltd.) as diluted with toluene (500 ml) was added thereto with stirring, followed by an aging reaction at 100°C for 24 hours.

After completion of the reaction, precipitated triethylamine hydrobromide was removed by filtration, and the obtained reaction solution was concentrated to obtain an ester of triethylenediamine (83.7 g). This ester of triethylenediamine was dissolved in tetrahydrofuran and added to a tetrahydrofuran solution of lithium aluminum hydride (17.1 g) under cooling with an ice bath with stirring.

After a reaction for 2 hours at room temperature, water (17 ml) and a 15 mass% sodium hydroxide aqueous solution (17 ml) were added to stop the reaction, and insolubles were removed by filtration.

The reaction solution was concentrated, and then 2-hydroxyalkyltriethylenediamine as a product was extracted and washed with ethyl acetate. Ethyl acetate was removed to obtain 48 g of 2-hydroxymethyltriethylenediamine (yield: 68%).

As is evident from the above Comparative Example 15, the process disclosed in Patent Document 1 requires

multistage reactions and thus was very cumbersome.

Now, the catalyst composition for the production of a polyurethane resin, containing the hydroxyalkyltriethylenediamine of the present invention, and a process for producing a polyurethane resin, employing such a composition, will be described in further detail with reference to the following Examples, but it should be understood that the present invention is by no means thereby restricted.

EXAMPLE 26

A polyol, a cell opener, a crosslinking agent, a surfactant and water were mixed in the raw material blend ratio as shown in Table 1 to prepare a premix A. 148.1 g of the premix A was taken into a 500 ml polyethylene cup, and as catalysts, 2-hydroxymethyltriethylenediamine and N,N,N'-trimethyl-N'-(2-hydroxyethyl)bis(2-aminoethyl)ether were added in a blend ratio shown in Table 2 (represented by gram), followed by adjusting the temperature to 20°C. An isocyanate liquid having the temperature adjusted to 20°C in a separate container was put into the cup of the premix A in such an amount that the isocyanate index [=isocyanate groups/OH groups (molar ratio) × 100] would become 100, followed by quickly stirring for 5 seconds by a stirring machine at 6,000 rpm. The mixed and stirred liquid was transferred to a 2 liter polyethylene cup having the temperature adjusted to 60°C, whereby the reactivity during blowing was measured. Further, from the obtained molded foam, the foam density was measured and compared. The results are shown in Table 2.

The methods for measuring the respective measuring items in Table 2 are as follows.

(1) Measuring items for the reactivity

Cream time: The blowing initiation time i.e. the time for initiation of rising of foam, was visually measured.
Gel time: As the reaction proceeds, the time for changing of a liquid substance to a resinous substance was measured.
Rise time: The time for stopping of the rising of foam was visually measured.

(2) Foam core density

The center portion of a molded foam was cut out in a size of 7 cm × 7 cm × 5 cm, and the size and weight were accurately measured, whereupon the core density was calculated.

(3) Odor of foam

From the foam, of which the foam core density was measured, a foam in a size of 5 cm × 5 cm × 5 cm was cut out and put in a mayonnaise bottle, which was then capped. This bottle was heated at 80°C for 1 hour, and then, the bottle was cooled to room temperature, whereupon the odor of the foam was smelled by 10 monitors, and the strength of the odor was measured.

◎: No substantial odor, ○: slight odor, Δ; substantial odor, ×: strong odor

TABLE 1

| | Parts by weight (pbw) |
|---|---|
| Premix A | |
| Polyol 1) | 92.6 |
| Cell opener 2) | 1.9 |
| Diethanolamine 3) | 0.7 |
| Silicon surfactant 4) | 1.0 |
| Water | 3.2 |
| 1) FA-703, polyether polyol (manufactured by Sanyo Chemical Industries, Ltd., OH value=34 mgKOH/g)<br>2) Voranol-1421 (manufactured by Dow Chemical)<br>3) Crosslinking agent (manufactured Aldrich)<br>4) Tegostab B4113LF (manufactured by Evonik) | |

TABLE 2

| | Ex. 26 | Ex. 27 | Ex. 28 | Ex. 29 | Ex. 30 | Ex. 31 | Comp. Ex. 16 | Comp. Ex. 17 |
|---|---|---|---|---|---|---|---|---|
| Amounts (pbw) | | | | | | | | |
| Premix A | 148.1 | 148.1 | 148.1 | 148.1 | 148.1 | 148.1 | 148.1 | 148.1 |
| 2-Hydroxymethyltriethylenediamine 1) | 0.60 | 0.60 | 0.60 | 0.60 | 0.60 | 0.60 | | |
| Amine compound A 2) | 0.15 | | | | | | | |
| Amine compound B 3) | | 0.15 | | | | | | 1.64 |
| Amine compound C 4) | | | 0.15 | | | | | |
| Amine compound D 5) | | | | 0.15 | | | | |
| Amine compound E 6) | | | | | 0.15 | | | |
| Amine compound F 7) | | | | | | 0.15 | | |
| TEDA-L33 8) | | | | | | | 0.48 | |
| TOYOCAT-ET 9) | | | | | | | 0.12 | |
| Isocyanate 10) | | | | | | | | |
| INDEX 11) | 100 | 100 | 100 | 100 | 100 | 100 | 100 | 100 |
| Reactivity (seconds) | | | | | | | | |
| Cream time | 12 | 13 | 14 | 15 | 14 | 15 | 10 | 9 |
| Gel time | 60 | 60 | 59 | 60 | 60 | 60 | 60 | 59 |
| Rise time | 80 | 86 | 85 | 84 | 85 | 78 | 85 | 84 |
| Core density (kg/m$^3$) | 38.1 | 37.3 | 37.5 | 37.4 | 37.3 | 38.6 | 38.8 | 37.5 |
| Odor of foam | ◎ | ○ | ○ | ◎ | ◎ | ◎ | × | × |

1) Amine product prepared in Comparative Example 15
2) N,N,N'-Trimethyl-N'-(2-hydroxyethyl)bis(2-aminoethyl) ether (manufactured by Tokyo Chemical Industry Co., Ltd.)
3) N,N-Dimethylaminoethanol (manufactured by Aldrich)
4) bis(3-Dimethylaminopropyl)amine (manufactured by Aldrich)
5) N,N-bis(3-Dimethylaminopropyl)-N-isopropanolamine (a product prepared by reacting propylene oxide to the amine compound C)
6) N,N-Dimethylaminohexanol (manufactured by Tokyo Chemical Industry Co., Ltd.)
7) N,N-Dimethyl-N',N'-bis(2-hydroxypropyl)-1,3-propanediamine (manufactured by Aldrich)
8) Dipropylene glycol solution containing 33.3 mass% of triethylenediamine (TEDA) (manufactured by TOSOH CORP. TEDA-L33)
9) Dipropylene glycol solution containing 70 mass% of bis(dimethylaminoethyl) ether (manufactured by TOSOH CORP. TOYOCAT-ET)
10) Coronate 1106 (manufactured by Nippon Polyurethane Industry Co., Ltd.)
11) INDEX=(mols of NCO groups/mols of OH group)×100

EXAMPLES 27 to 31

A foam was formed in the same manner as in Example 26 except that the amine compound shown in Table 2 was used instead of N,N,N'-trimethyl-N'-(2-hydroxyethyl)bis(2-aminoethyl) ether. The results are also shown in Table 2.

COMPARATIVE EXAMPLE 16

A foam was formed in the same manner as in Example 26 except that triethylenediamine (manufactured by TOSOH CORPORATION, tradename: TEDA-L33) and a dipropylene glycol solution containing 70% of bis(dimethylaminoethyl)ether (manufactured by TOSOH CORPORATION, tradename: TOYOCAT-ET) were used instead of 2-hydroxymethyltriethylenediamine and N,N,N'-trimethyl-N'-(2-hydroxyethyl)bis(2-aminoethyl) ether. The results are also shown in Table 2.

COMPARATIVE EXAMPLE 17

A foam was formed in the same manner as in Example 26 except that N,N-dimethylaminoethanol was used instead of 2-hydroxymethyltriethylenediamine and N,N,N'-trimethyl-N'-(2-hydroxyethyl)bis(2-aminoethyl) ether. The results are also shown in Table 2.

Examples 26 to 31 are examples wherein the catalyst compositions of the present invention were used, whereby the catalytic activities were high, and from the foam, the odor of the amine catalyst was not substantially identified. In a case where TEDA-L33 and TOYOCAT-ET which are commonly used as urethane catalysts, were used (Comparative Example 16), the odor of the amine catalyst from the foam was confirmed, and further, it was not possible to prevent a fogging phenomenon of a window glass or discoloration of PVC of an instrument panel of an automobile attributable to the amine catalyst.

On the other hand, in a case where N,N-dimethylaminoethanol as a reactive catalyst was used alone (Comparative Example 17), the catalyst activities were low, and the odor of the amine catalyst from the foam was confirmed, and it was not possible to prevent a fogging phenomenon of a window glass or discoloration of PVC of an instrument panel of an automobile attributable to the amine catalyst.

(Calculation of gelling reaction rate constant)

REFERENCE EXAMPLE 1

Into a 200 ml three-necked flask purged with nitrogen, 50 ml of a DEG-containing benzene solution prepared to have a diethylene glycol (DEG) concentration of 0.15 mol/L was taken, and 60.7 mg (0.35 mmol) of N,N,N',N",N"-pentamethyldiethylenetriamine (manufactured by TOSOH CORPORATION, tradename: TOYOCAT-DT) was added thereto to obtain liquid A.

Then, into a 100 ml three-necked flask purged with nitrogen, 50 ml of a TDI-containing benzene solution prepared to have a 2,6-toluenediisocyaante (TDI) concentration of 0.15 mol/L, was taken and designated as liquid B.

The liquids A and B were, respectively, held at 30°C for 30 minutes, and then, the liquid B was added to the liquid A to initiate a reaction with stirring. After initiation of the reaction, every 10 minutes, about 10 ml of the reaction solution was taken, and an unreacted isocyanate was reacted with an excess di-n-butylamine (DBA) solution, and the remaining DBA was back-titrated with 0.2 N hydrochloric acid ethanol solution to quantify the amount of the unreacted isocyanate.

As mentioned above, the reaction rate constant k (L/mol·h) was obtained on an assumption that the reaction (gelling reaction) of an isocyanate with an alcohol is linear to the respective concentrations. Further, the rate constant Kc ($L^2$/eq·mol·h) corresponding to each catalyst was obtained by dividing the reaction rate constant k by the catalyst concentration. Further, the gelling reaction rate constant k1w ($L^2$/g·mol· h) which can be regarded as an active power per weight, was obtained by dividing Kc by the molecular weight of the catalyst. The results are shown in Table 3.

TABLE 3

| | Reference Examples | | | | | | | | | | | | | | | |
|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 |
| Catalyst (mmol) | | | | | | | | | | | | | | | | |
| TOYOCAT-DT 1) | 0.35 | | | | | | | | 0.35 | | | | | | | |
| TOYOCAT-ET 2) | | 0.35 | | | | | | | | 0.35 | | | | | | |
| TOYOCAT-RX5 3) | | | 0.35 | | | | | | | | 0.35 | | | | | |
| Amine compound A 4) | | | | 0.35 | | | | | | | | 0.35 | | | | |
| Amine compound B 5) | | | | | 0.35 | | | | | | | | 0.35 | | | |
| Amine compound C 6) | | | | | | 0.35 | | | | | | | | 0.35 | | |
| Amine compound D 7) | | | | | | | 0.35 | | | | | | | | 0.35 | |
| TOYOCAT-MR 8) | | | | | | | | 0.35 | | | | | | | | 0.35 |
| DEG (mmol) 9) | 7.50 | 7.50 | 7.50 | 7.50 | 7.50 | 7.50 | 7.50 | 7.50 | | | | | | | | |
| Water (mmol) | | | | | | | | | 7.80 | 7.80 | 7.80 | 7.80 | 7.80 | 7.80 | 7.80 | 7.80 |
| Isocyanate (mmol) 10) | | | | | | | | | | | | | | | | |
| TDI | 7.50 | 7.50 | 7.50 | 7.50 | 7.50 | 7.50 | 7.50 | 7.50 | 7.80 | 7.80 | 7.80 | 7.80 | 7.80 | 7.80 | 7.80 | 7.80 |
| Reaction rate constant ($L^2/g \cdot mol \cdot h$) | | | | | | | | | | | | | | | | |
| k1w (gelling) | 0.43 | 0.21 | 0.29 | 0.18 | 0.37 | 0.34 | 0.29 | 0.30 | | | | | | | | |
| K2w (blowing) | | | | | | | | | 1.59 | 0.82 | 0.43 | 0.26 | 1.05 | 0.89 | 0.036 | 0.084 |

1) N,N,N',N",N"-Pentamethyldiethylenetriamine (manufactured by TOSOH CORPORATION, TOYOCAT-DT)
2) Dipropylene glycol solution containing 70 mass% of bis(dimethylaminoethyl) ether (manufactured by TOSOH CORPORATION, tradename: TOYOCAT-ET)
3) N,N,N'-Trimethylaminoethylethanolamine (manufactured by TOSOH CORPORATION, tradename: TOYOCAT-RX5)
4) N,N-Dimethylaminoethoxyethanol (manufactured by Aldrich)
5) Hexamethyltriethylenetetramine (product prepared by reacting triethylenetetramine with formalin for reduction methylation)
6) N,N,N'-Trimethyl-N'-(2-hydroxyethyl)bis(2-aminoethyl) ether (manufactured by Tokyo Chemical Industry Co., Ltd.)
7) N,N-Dimethylaminoethanol (manufactured by Aldrich)
8) N,N,N',N'-Tetramethylhexamethylenediamine (manufactured by TOSOH CORPORATION, tradename: TOYOCAT-MR)
9) Diethylene glycol (manufactured by Kishida Chemical Co., Ltd.)
10) 2,6-Toluenediisocyanate (manufactured by Tokyo Chemical Industry Co., Ltd.)

REFERENCE EXAMPLES 2 to 8

The gelling reaction rate constant k1w was calculated in the same manner as in Reference Example 1 except that the tertiary amine compound shown in Table 3 was used as the catalyst. The results are also shown in Table 3.

(Calculation of blowing reaction rate constant)

REFERENCE EXAMPLE 9

Into a 200 ml three-necked flask purged with nitrogen, 100 ml of water-containing benzene solution prepared to have a water concentration of 0.078 mol/L was taken, and 60.7 mg (0.35 mmol) of N,N,N',N",N"-pentamethyldiethylenetriamine (manufactured to TOSOH CORPORATION, tradename: TOYOCAT-DT) was added thereto to obtain liquid A.

Then, into a 100 ml three-necked flask purged with nitrogen, 10 ml of a TDI-containing benzene solution prepared to have a 2,6-toluenediisocyaante (TDI) concentration of 0.78 mol/L, was taken and designated as liquid B.

The liquids A and B were, respectively, held at 30°C for 30 minutes, and then, the liquid B was added to the liquid A to initiate a reaction with stirring. After initiation of the reaction, every 10 minutes, about 10 ml of the reaction solution was taken, and an unreacted isocyanate was reacted with an excess di-n-butylamine (DBA) solution, and the remaining DBA was back-titrated with 0.2 N hydrochloric acid ethanol solution to quantify the amount of the unreacted isocyanate.

As mentioned above, the reaction rate constant k (L/mol·h) was obtained on an assumption that the reaction (blowing reaction) of an isocyanate with water is linear to the respective concentrations. Further, the rate constant Kc ($L^2$/eq·mol·h) corresponding to each catalyst was obtained by dividing the reaction rate constant k by the catalyst concentration. Further, k2w ($L^2$/g·mol·h) which can be regarded as an active power per weight, was obtained by dividing Kc by the molecular weight of the catalyst. The results are shown in Table 3.

REFERENCE EXAMPLES 10 to 16

The blowing reaction rate constant k2w was calculated in the same manner as in Reference Example 9 except that the tertiary amine compound shown in Table 3 was used as the catalyst. The results are also shown in Table 3.

(Calculation of blowing/gelling activity ratio)

From the results in Table 3, the blowing/gelling activity ratio (=[gelling reaction rate constant k1w/blowing reaction rate constant k2w]) of the tertiary amine compound was obtained. The results are summarized in Table 4.

TABLE 4

| | Reaction rate constant ($L^2$/g·mol·h) | | Blowing/gelling activity ratio k2w/k1w |
|---|---|---|---|
| | k1w (gelling) | k2w (blowing) | |
| TOYOCAT-DT 1) | 0.43 | 1.59 | 3.73 |
| TOYOCAT-ET 2) | 0.21 | 0.82 | 3.92 |
| TOYOCAT-RX5 3) | 0.29 | 0.43 | 1.50 |
| Amine compound A 4) | 0.18 | 0.26 | 1.39 |
| Amine compound B 5) | 0.37 | 1.05 | 2.85 |
| Amine compound C 6) | 0.34 | 0.89 | 2.59 |
| Amine compound D 7) | 0.29 | 0.036 | 0.12 |

(continued)

| | Reaction rate constant ($L^2$/g·mol·h) | | Blowing/gelling activity ratio k2w/k1w |
|---|---|---|---|
| | k1w (gelling) | k2w (blowing) | |
| TOYOCAT-MR 8) | 0.30 | 0.084 | 0.28 |

1) N,N,N',N",N"-Pentamethyldiethylenetriamine (manufactured by TOSOH CORPORATION, TOYOCAT-DT)
2) Dipropylene glycol solution containing 70 mass% of bis(dimethylaminoethyl) ether (manufactured by TOSOH CORPORATION, tradename: TOYOCAT-ET)
3) N,N,N'-Trimethylaminoethylethanolamine (manufactured by TOSOH CORPORATION, tradename: TOYOCAT-RX5)
4) N,N-Dimethylaminoethoxyethanol (manufactured by Aldrich)
5) Hexamethyltriethylenetetramine (product prepared by reacting triethylenetetramine with formalin for reduction methylation)
6) N,N,N'-Trimethyl-N'-(2-hydroxyethyl)bis(2-aminoethyl) ether (manufactured by Tokyo Chemical Industry Co., Ltd.)
7) N,N-Dimethylaminoethanol (manufactured by Aldrich)
8) N,N,N',N'-Tetramethylhexamethylenediamine (manufactured by TOSOH CORPORATION, tradename: TOYOCAT-MR)

EXAMPLE 32 - not forming part of the claimed invention

A polyol, a cell opener, a crosslinking agent, a surfactant and water were mixed in the raw material blend ratio as shown in Table 5 to obtain a premix A.

148.1 g of the premix A was taken into a 500 ml polyethylene cup, and as catalysts, 2-hydroxymethyltriethylenediamine prepared in Comparative Example 15 and the dipropylene glycol solution containing 70 mass% of bis(dimethylaminoethyl) ether (manufactured by TOSOH CORPORATION, tradename: TOYOCAT-ET) were added in the blend ratio as shown in Table 6 (represented by g), and the temperature was adjusted to 20°C.

An isocyanate liquid having the temperature adjusted to 20°C in a separate container was put into the cup of the premix A in such an amount that the isocyanate index [=isocyanate groups/OH groups (molar ratio) × 100] would become 100, followed by quickly stirring at 6,000 rpm for 5 seconds by a stirring machine.

The mixed and stirred liquid was transferred to a 2 liter polyethylene cup having the temperature adjusted to 60°C, whereby the reactivity during blowing was measured. Further, from the obtained molded foam, the foam density was measured and compared. The results are shown in Table 6.

Here, the measuring methods for the respective measuring items, such as (1) the measuring items for the reactivity, (2) the foam core density and (3) odor of the foam in Table 6 were the same as the measuring methods in Table 2.

TABLE 5

| | Parts by weight (pbw) |
|---|---|
| Premix A | |
| Polyol 1) | 92.6 |
| Cell opener 2) | 1.9 |
| Diethanolamine 3) | 0.7 |
| Silicon surfactant 4) | 1.0 |
| Water | 3.2 |

1) FA-703, polyether polyol (manufactured by Sanyo Chemical Industries, Ltd., OH value=34 mgKOH/g)
2) Voranol-1421 (manufactured by Dow Chemical)
3) Crosslinking agent (manufactured Aldrich)
4) Tegostab B4113LF (manufactured by Evonik)

TABLE 6

| | Activity ratio[1)] | Examples | | | | | | | | | | | |
|---|---|---|---|---|---|---|---|---|---|---|---|---|---|
| | | 32 | 33 | 34 | 35 | 36 | 37 | 38 | 39 | 40 | 41 | 42 | 43 |
| Amounts (pbw) | | | | | | | | | | | | | |
| Premix A | | 148.1 | 148.1 | 148.1 | 148.1 | 148.1 | 148.1 | 148.1 | 148.1 | 148.1 | 148.1 | 148.1 | 148.1 |
| 2-Hydroxymethyltriethylenediamine [2)] | | 0.75 | 0.69 | 0.43 | 0.24 | 0.60 | 1.23 | 1.30 | 0.90 | 1.09 | 0.97 | 0.64 | 0.39 |
| TOYOCAT-ET [3)] | 3.92 | 0.15 | 0.17 | 0.22 | 0.24 | | | | | | | | |
| TOYOCAT-DT [4)] | 3.73 | | | | | 0.15 | | | | | | | |
| TOYOCAT-RX5 [5)] | 1.50 | | | | | | 0.31 | | | | | | |
| Amine compound A [6)] | 1.39 | | | | | | | 0.32 | | | | | |
| Amine compound B [7)] | 2.85 | | | | | | | | 0.23 | | | | |
| Amine compound C [8)] | 2.59 | | | | | | | | | 0.22 | 0.24 | 0.32 | 0.39 |
| Isocyanate [9)] | | | | | | | | | | | | | |
| INDEX [10)] | | 100 | 100 | 100 | 100 | 100 | 100 | 100 | 100 | 100 | 100 | 100 | 100 |
| Reactivity (seconds) | | | | | | | | | | | | | |
| Cream time | | 12 | 10 | 9 | 8 | 11 | 15 | 15 | 12 | 13 | 14 | 12 | 11 |
| Gel time | | 62 | 61 | 61 | 61 | 63 | 62 | 63 | 62 | 62 | 63 | 61 | 62 |
| Rise time | | 80 | 76 | 70 | 63 | 80 | 85 | 88 | 82 | 87 | 83 | 80 | 76 |
| Core density ($kg/m^3$) | | 39.2 | 39.6 | 43.0 | 46.2 | 39.8 | 37.7 | 37.4 | 39.5 | 38.6 | 37.6 | 39.3 | 41.0 |
| Odor of foam | | ◎ | ◎ | ○ | ○ | ◎ | ◎ | ◎ | ○ | ◎ | ◎ | ◎ | ◎ |

1) Blowing/gelling activity ratio (=[blowing reaction rate constant/gelling reaction rate constant]) shown in Table 4
2) Amine product prepared in Comparative Example 15
3) Dipropylene glycol solution containing 70 mass% of bis(dimethylaminoethyl ether (manufactured by TOSOH CORP. TOYOCAT-ET)
4) N,N,N',N",N"-Pentamethyldiethylenetriamine (manufactured by TOSOH CORPORATION, tradename: TOYOCAT-DT)
5) N,N,N'-Trimethylaminoethylethanolamine (manufactured by TOSOH CORPORATION, tradename: TOYOCAT-RX5)
6) N,N-Dimethylaminoethoxyethanol (manufactured by Tokyo Aldrich)
7) Hexamethyltriethylenetetramine (product prepared by reacting triethylenetetramine with formalin for reduction methylation)
8) N,N,N'-Trimethyl-N'-(2-hydroxyethyl)bis(2-aminoethyl) ether (manufactured by Tokyo Chemical Industry Co., Ltd.)
9) Coronate 116 (manufactured by Nippon Polyurethane Industry Co., Ltd.)
10) Isocyanate INDEX=(mols of NCO groups/mols of OH group)×100

EXAMPLES 33 to 43 - not forming part of the claimed invention

A foam was formed in the same manner as in Example 32 except that instead of bis(dimethylaminoethyl) ether, the amine compound shown in Table 6 was used. The results are also shown in Table 6.

Examples 32 to 43 are Examples not forming part of the claimed invention in which the catalyst compositions of the present invention were used, and as is evident from Table 6, the catalytic activity is high in each case, and the odor of the amine catalyst was not substantially identified from the obtained foam.

COMPARATIVE EXAMPLES 18 and 19

A foam was formed in the same manner as in Example 32 except that instead of bis(dimethylaminoethyl) ether, the amine compound shown in Table 7 was used. The results are also shown in Table 7.

TABLE 7

| | Activity ratio1) | Comparative Examples | | | | | |
|---|---|---|---|---|---|---|---|
| | | 18 | 19 | 20 | 21 | 22 | 23 |
| Amounts (pbw) | | | | | | | |
| Premix A | | 148.1 | 148.1 | 148.1 | 148.1 | 148.1 | 148.1 |
| 2-Hydroxymethyltriethylenediamine 2) | | 1.65 | 1.31 | 2.32 | | | |
| TOYOCAT-ET 3) | 3.92 | | | | 0.12 | 0.17 | 0.21 |
| Amine compound D 4) | 0.12 | 0.41 | | | | | |
| TOYOCAT-MR 5) | 0.28 | | 0.33 | | | | |
| TEDA-L33 6) | | | | | 0.50 | 0.35 | 0.21 |
| Isocyanate 7) | | | | | | | |
| INDEX 8) | | 100 | 100 | 100 | 100 | 100 | 100 |
| Reactivity (seconds) | | | | | | | |
| Cream time | | 13 | 13 | 18 | 12 | 11 | 11 |
| Gel time | | 61 | 60 | 62 | 62 | 62 | 62 |
| Rise time | | 90 | 85 | 91 | 85 | 78 | 70 |
| Core density (kg/m$^3$) | | 37.6 | 37.7 | 38.0 | 38.6 | 40.1 | 43.8 |
| Odor of foam | | × | × | ○ | × | × | × |

1) Blowing/gelling activity ratio (=[blowing reaction rate constant/gelling reaction rate constant]) shown in Table 4
2) Amine product prepared in Comparative Example 15
3) Dipropylene glycol solution containing 70 mass% of bis(dimethylaminoethyl) ether (manufactured by TOSOH CORP. TOYOCAT-ET)
4) N,N-Dimethylaminoethanol (manufactured by Aldrich)
5) N,N,N',N'-Tetramethylhexanediamine (manufactured by TOSOH CORPORATION, tradename: TOYOCAT-MR)
6) Dipropylene glycol solution containing 33.3 mass% of triethylenediamine (manufactured by TOSOH CORP. TEDA-L33)
7) Coronate 116 (manufactured by Nippon Polyurethane Industry Co., Ltd.)
8) Isocyanate INDEX=(mols of NCO groups/mols of OH group)×100

COMPARATIVE EXAMPLE 20

A foam was formed in the same manner as in Example 32 except that as the catalyst, 2-hydroxymethyltriethylenediamine prepared in Comparative Example 15 was used alone. The results are also shown in Table 7.

COMPARATIVE EXAMPLES 21 to 23

A foam was formed n the same manner as in Example 32 except that instead of 2-hydroxymethyltriethylenediamine, triethylenediamine (manufactured by TOSOH CORPORATION, tradename: TEDA-L33) was used. The results are also shown in Table 7.

As is evident from Table 7, in a case where as a tertiary amine compound, one having a value of [blowing reaction

rate constant/gelling reaction rate constant] of smaller than 0.5, was used (Comparative Examples 18 and 19), the amount of the catalyst used increased, and an odor of the amine catalyst from the foam was confirmed. Accordingly, it was not possible to prevent a fogging phenomenon of the window glass or discoloration of PVC of an instrument panel of an automobile attributable to the amine catalyst.

Further, in a case where 2-hydroxymethyltriethylenediamine was used alone (Comparative Example 20), although it was possible to reduce the odor of the amine catalyst from the obtained foam, the cream time was slow, and it was not possible to form the foam with good productivity.

On the other hand, in cases wherein 2-hydroxymethyltriethylenediamine was not used, and the dipropylene glycol solution containing 33.3 mass% of triethylenediamine (manufactured by TOSOH CORPORATION, TEDA-L33) and the dipropylene glycol solution containing 70 mass% of bis(dimethylaminoethyl) ether (manufactured by TOSOH CORPORATION, tradename: TOYOCAT-ET) which are commonly used, were used (Comparative Examples 21 to 23), an odor of the amine compound from the foam was confirmed, and it was not possible to prevent a fogging phenomenon of a window glass or discoloration of PVC of an instrument panel for an automobile attributable to the amine catalyst.

INDUSTRIAL APPLICABILITY

The process for producing a hydroxyalkyltriethylenediamine or hydroxytriethylenediamine not forming part of the present invention requires no multistage reaction steps and is simple with a small number of steps, and the process for producing a polyurethane resin employing a catalyst composition containing such a diamine is capable of producing a polyurethane product with good productivity and good moldability without bringing about odor problems or environmental problems, such being industrially advantageous.

Reference is made to Japanese Patent Application No. 2008-142586 filed on May 30, 2008, Japanese Patent Application No. 2008-178990 filed on July 9, 2008, Japanese Patent Application No. 2008-185165 filed on July 16, 2008, Japanese Patent Application No. 2008-204535 filed on August 7, 2008, Japanese Patent Application No. 2008-278254 filed on October 29, 2008, Japanese Patent Application No. 2008-281558 filed on October 31, 2008, Japanese Patent Application No. 2008-296910 filed on November 20, 2008 and Japanese Patent Application No. 2008-297912 filed on November 21, 2008 including specifications, claims and summaries..

## Claims

**1.** A catalyst composition for the production of a polyurethane resin, which comprises :

a hydroxyalkyltriethylenediamine or hydroxytriethylenediamine (A), wherein the hydroxyalkyltriethylenediamine or hydroxytriethylenediamine (A) is one or more selected from the group consisting of
a hydroxyalkyltriethylenediamine or hydroxytriethylenediamine represented by the following formula (2a):

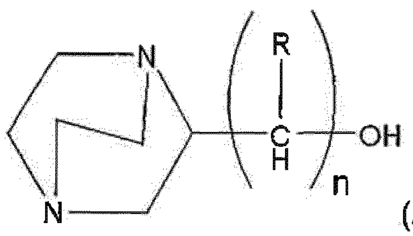

(2a)

where in the above formula (2a), R is a hydrogen atom or a linear or branched $C_{1-4}$ alkyl group, and n is an integer of from 0 to 6,
a hydroxyalkyltriethylenediamine represented by the following formula (2b):

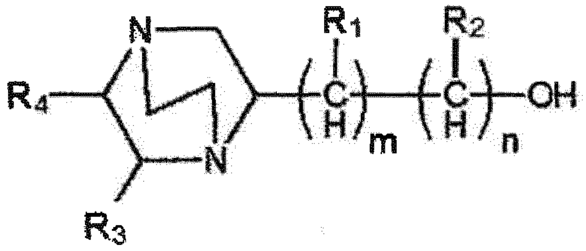

(2b)

where in the above formula (2b), each of $R_1$ to $R_4$ which are independent of each other, is a hydrogen atom or a linear or branched $C_{1-4}$ alkyl group, and each of m and n which are independent of each other, is an integer of from 0 to 2, provided m+n < 4, and/or

a hydroxyalkyltriethylenediamine represented by the following formula (2c):

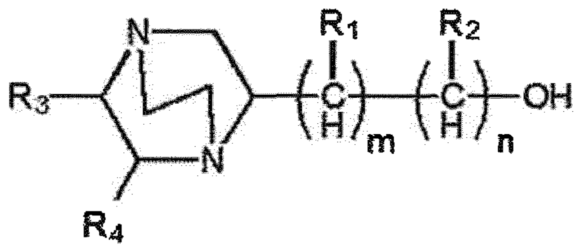

(2c)

where in the above formula (2c), each of $R_1$ to $R_4$ which are independent of each other, is a hydrogen atom or a linear or branched $C_{1-4}$ alkyl group, and each of m and n which are independent of each other, is an integer of from 0 to 2, provided m+n < 4, and

a hydroxymethyltriethylenediamine represented by the following formula (2d):

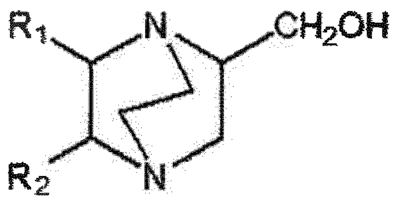

(2d)

where in the above formula (2d), each of $R_1$ and $R_2$ which are independent of each other, is a hydrogen atom or a $C_{1-4}$ alkyl group; and

an amine compound (B) having, in its molecule, one or more substituents selected from the group consisting of a hydroxy group, a primary amino group and a secondary amino group,

wherein the amine compound (B) having, in its molecule, one or more substituents selected from the group consisting of a hydroxy group, a primary amino group and a secondary amino group, is an amine compound represented by the following formula (11):

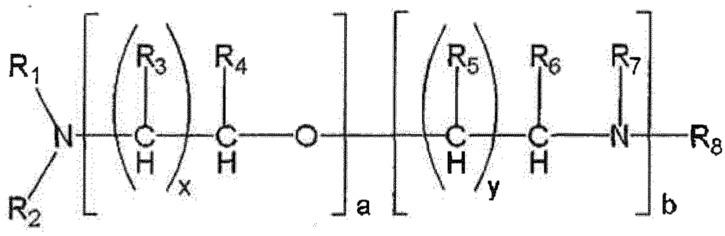

(11)

where in the above formula (11), each of $R_1$ to $R_8$ which are independent of each other, is a hydrogen atom, a

hydroxyl group, a $C_{1-16}$ alkyl group, a $C_{6-16}$ aryl group, a $C_{1-10}$ hydroxyalkyl group, a $C_{1-10}$ aminoalkyl group, a $C_{1-10}$
monomethylaminoalkyl group or a $C_{1-10}$ dimethylaminoalkyl group, x is an integer of from 0 to 11, y is an integer of from 0 to 11, a is an integer of from 0 to 10 and b is an integer of from 0 to 10, and
wherein the amine compound represented by the above formula (11) is one or more amines selected from the group consisting of N,N-dimethylethylenediamine, N,N'-dimethylethylenediamine, N,N-dimethylpropylenediamine, N,N'-dimethylpropylenediamine, N,N-dimethylhexamethylenediamine, N,N'-dimethylhexamethylenediamine, trimethyldiethylenetriamine, trimethylethylenediamine, trimethylpropylenediamine, trimethylhexamethylenediamine, tetramethyldiethylenetriamine, N,N-dimethylaminoethanol, N,N-dimethylaminoisopropanol, bis(3-dimethylaminopropyl)amine, N-methylpiperazine, N,N-dimethylaminoethoxyethanol, N,N,N'-trimethylaminoethylethanolamine, N,N-dimethylaminoethyl-N'-methylaminoethyl-N"-methylaminoisopropanol, N,N-dimethylaminoethoxyethoxyethanol, N,N-dimethyl-N',N'-bis(2-hydroxypropyl)-1,3-propanediamine, N,N,N'-trimethyl-N'-(2-hydroxyethyl)bis(2-aminoethyl) ether, N,N-bis(3-dimethylaminopropyl)-N-isopropanolamine, N,N-dimethylaminohexanol and N,N,N'-trimethyl-N'-(2-hydroxyethyl)propylenediamine.

**2.** The catalyst composition for the production of a polyurethane resin according to Claim 1, wherein the hydroxyalkyltriethylenediamine or hydroxytriethylenediamine (A) is an amine compound represented by the following formula (2e):

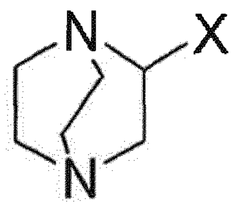

(2e)

where in the above formula (2e), X is a hydroxyl group, a hydroxymethyl group or a hydroxyethyl group.

**3.** The catalyst composition for the production of a polyurethane resin according to Claim 1 or 2, wherein the mixed ratio of the hydroxyalkyltriethylenediamine or hydroxytriethylenediamine (A), to the amine compound (B) having, in its molecule, one or more substituents selected from the group consisting of a hydroxy group, a primary amino group and a secondary amino group, is amine compound (A)] / amine compound (B) = 1/99 to 99/1 weight ratio.

**4.** A process for producing a polyurethane resin, which comprises reacting a polyol with a polyisocyanate in the presence of the catalyst composition as defined in any one of Claims 1 to 3.

**5.** The process for producing a polyurethane resin according to Claim 4,
wherein the catalyst composition as defined in any one of Claims 1 to 3, is used in an amount within a range of from 0.01 to 30 parts by weight per 100 parts by weight of the polyol.

**Patentansprüche**

**1.** Katalysatorzusammensetzung zur Herstellung eines Polyurethanharzes, die umfasst:
ein Hydroxyalkyltriethylendiamin oder Hydroxytriethylendiamin (A), wobei das Hydroxyalkyltriethylendiamin oder Hydroxytriethylendiamin (A) eines oder mehrere ist bzw. sind, das bzw. die ausgewählt ist bzw. sind aus der Gruppe bestehend aus:

einem Hydroxyalkyltriethylendiamin oder Hydroxytriethylendiamin, dargestellt durch die nachstehende Formel (2a):

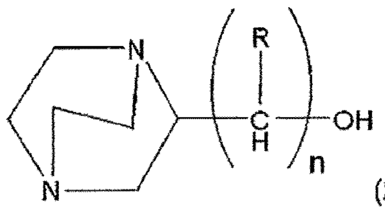

(2a)

wobei in der obigen Formel (2a) R ein Wasserstoffatom oder eine lineare oder verzweigte $C_{1-4}$-Alkylgruppe ist und n eine ganze Zahl von 0 bis 6 ist,
einem Hydroxyalkyltriethylendiamin, dargestellt durch die nachstehende Formel (2b):

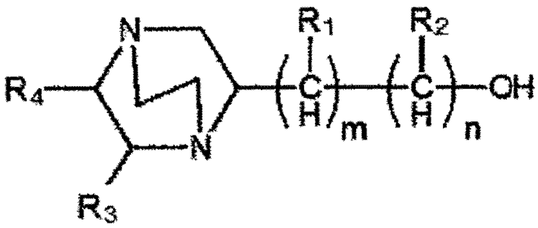

(2b)

wobei in der obigen Formel (2b) jedes von $R_1$ bis $R_4$, die voneinander unabhängig sind, ein Wasserstoffatom oder eine lineare oder verzweigte $C_{1-4}$-Alkylgruppe ist und jedes von m und n, die voneinander unabhängig sind, eine ganze Zahl von 0 bis 2 ist, vorausgesetzt m+n < 4, und/oder
einem Hydroxyalkyltriethylendiamin, dargestellt durch die nachstehende Formel (2c):

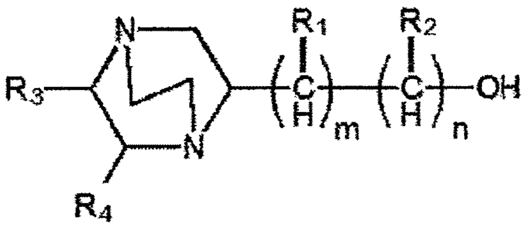

(2c)

wobei in der obigen Formel (2c) jedes von $R_1$ bis $R_4$, die voneinander unabhängig sind, ein Wasserstoffatom oder eine lineare oder verzweigte $C_{1-4}$-Alkylgruppe ist und jedes von m und n, die voneinander unabhängig sind, eine ganze Zahl von 0 bis 2 ist, vorausgesetzt m+n < 4, und
einem Hydroxymethyltriethylendiamin, dargestellt durch die nachstehende Formel (2d):

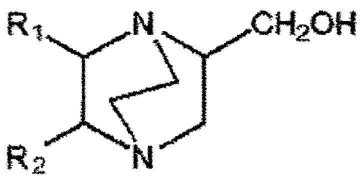

(2d)

wobei in der obigen Formel (2d) jedes von $R_1$ und $R_2$, die voneinander unabhängig sind, ein Wasserstoffatom oder eine $C_{1-4}$-Alkylgruppe ist; und
eine Aminverbindung (B), die in ihrem Molekül, einen oder mehrere Substituenten umfasst, die ausgewählt sind aus der Gruppe bestehend aus einer Hydroxygruppe, einer primären Aminogruppe und einer sekundären Aminogruppe,
wobei die Aminverbindung (B), die, in ihrem Molekül, einen oder mehrere Substituenten umfasst, die ausgewählt sind aus der Gruppe bestehend aus einer Hydroxygruppe, einer primären Aminogruppe und einer sekundären Aminogruppe, eine Aminverbindung ist, dargestellt durch die nachstehende Formel (11):

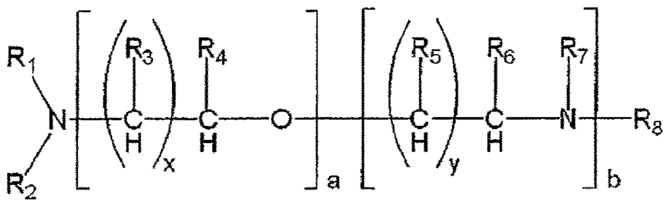

(11)

wobei in der obigen Formel (11) jedes von $R_1$ bis $R_8$, die voneinander unabhängig sind, ein Wasserstoffatom, eine Hydroxylgruppe, eine $C_{1-16}$-Alkylgruppe, eine $C_{6-16}$-Arylgruppe, eine $C_{1-10}$-Hydroxyalkylgruppe, eine $C_{1-10}$-Aminoalkylgruppe, eine $C_{1-10}$-Monomethylaminoalkylgruppe oder eine $C_{1-10}$-Dimethylaminoalkylgruppe ist, x eine ganze Zahl von 0 bis 11 ist, y eine ganze Zahl von 0 bis 11 ist, a eine ganze Zahl von 0 bis 10 ist und b eine ganze Zahl von 0 bis 10 ist, und

wobei die Aminverbindung, dargestellt durch die vorstehende Formel (11), ein oder mehrere Amine sind, die ausgewählt sind aus der Gruppe bestehend aus N,N-Dimethylethylendiamin, N,N'-Dimethylethylendiamin, N,N-Dimethylpropylendiamin, N,N'-Dimethylpropylendiamin, N,N-Dimethylhexamethylendiamin, N,N'-Dimethylhexamethylendiamin, Trimethyldiethylentriamin, Trimethylethylendiamin, Trimethylpropylendiamin, Trimethylhexamethylendiamin, Tetramethyldiethylentriamin, N,N-Dimethylaminoethanol, N,N-Dimethylaminoisopropanol, Bis(3-dimethylaminopropyl)amin, N-Methylpiperazin, N,N-Dimethylaminoethoxyethanol, N,N,N'-Trimethylaminoethylethanolamin, N,N-Dimethylaminoethyl-N'-methylaminoethyl-N''-methylaminoisopropanol, N,N-Dimethylaminoethoxyethoxyethanol, N,N-Dimethyl-N',N'-bis(2-hydroxypropyl)-1,3-propandiamin, N,N,N'-Trimethyl-N'-(2-hydroxyethyl)bis(2-aminoethyl)ether, N,N-bis(3-Dimethylaminopropyl)-N-isopropanolamin, N,N-Dimethylaminohexanol und N,N,N'-Trimethyl-N'-(2-hydroxyethyl)propylendiamin.

2. Katalysatorzusammensetzung zur Herstellung eines Polyurethanharzes nach Anspruch 1, wobei das Hydroxyalkyltriethylendiamin oder Hydroxytriethylendiamin (A) eine Aminverbindung ist, dargestellt durch die nachstehende Formel (2e):

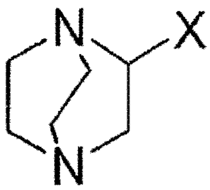

(2e)

wobei in der obigen Formel (2e) X eine Hydroxylgruppe, eine Hydroxymethylgruppe oder eine Hydroxyethylgruppe ist.

3. Katalysatorzusammensetzung zur Herstellung eines Polyurethanharzes nach Anspruch 1 oder 2, wobei das Mischungsverhältnis des Hydroxyalkyltriethylendiamins oder Hydroxytriethylendiamins (A) zur Aminverbindung (B), die in ihrem Molekül, einen oder mehrere Substituenten, die ausgewählt sind aus der Gruppe bestehend aus einer Hydroxygruppe, einer primären Aminogruppe und einer sekundären Aminogruppe, umfasst, das Gewichtsverhältnis Aminverbindung (A) /Aminverbindung (B) = 1/99 bis 99/1 ist.

4. Verfahren zur Herstellung eines Polyurethanharzes, umfassend das Reagieren eines Polyols mit einem Polyisocyanat bei Vorhandensein der nach einem der Ansprüche 1 bis 3 definierten Katalysatorzusammensetzung.

5. Verfahren zur Herstellung eines Polyurethanharzes nach Anspruch 4, wobei die nach einem der Ansprüche 1 bis 3 definierte Katalysatorzusammensetzung in einer Menge in einem Bereich von 0,01 bis zu 30 Gewichtsteilen je 100 Gewichtsteilen des Polyols verwendet wird.

**Revendications**

1. Composition de catalyseur pour la production d'une résine de polyuréthane, qui comprend :

une hydroxyalkyltriéthylènediamine ou hydroxytriéthylènediamine (A), laquelle hydroxyalkyltriéthylènediamine ou hydroxytriéthylènediamine (A) est une ou plusieurs choisies dans le groupe constitué par

une hydroxyalkyltriéthylènediamine ou hydroxytriéthylènediamine représentée par la formule (2a) suivante :

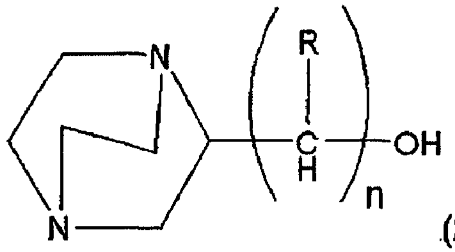

(2a)

où, dans la formule (2a) ci-dessus, R est un atome d'hydrogène ou un groupe alkyle en $C_1$ à $C_4$ linéaire ou ramifié, et n est un entier de 0 à 6,
une hydroxyalkyltriéthylènediamine représentée par la formule (2b) suivante :

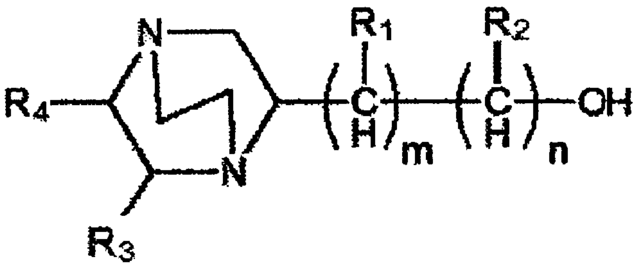

(2b)

où, dans la formule (2b) ci-dessus, chacun de $R_1$ à $R_4$, qui sont indépendants les uns des autres, est un atome d'hydrogène ou un groupe alkyle en $C_1$ à $C_4$ linéaire ou ramifié, et chacun de m et n, qui sont indépendants l'un de l'autre, est un entier de 0 à 2, sous réserve que m+n < 4, et/ou
une hydroxyalkyltriéthylènediamine représentée par la formule (2c) suivante :

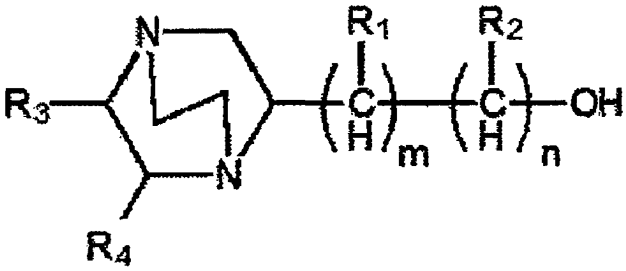

(2c)

où, dans la formule (2c) ci-dessus, chacun de $R_1$ à $R_4$, qui sont indépendants les uns des autres, est un atome d'hydrogène ou un groupe alkyle en $C_1$ à $C_4$ linéaire ou ramifié, et chacun de m et n, qui sont indépendants l'un de l'autre, est un entier de 0 à 2, sous réserve que m+n < 4, et
une hydroxyméthyltriéthylènediamine représentée par la formule (2d) suivante :

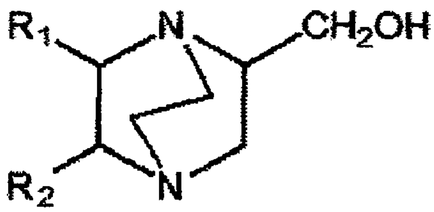

(2d)

où, dans la formule (2d) ci-dessus, chacun de $R_1$ et $R_2$, qui sont indépendants l'un de l'autre, est un atome

d'hydrogène ou un groupe alkyle en $C_1$ à $C_4$ ; et
un composé amine (B) ayant, dans sa molécule, un ou plusieurs substituants choisis dans l'ensemble constitué par un groupe hydroxy, un groupe amino primaire, et un groupe amino secondaire,
dans laquelle le composé amine (B) ayant, dans sa molécule, un ou plusieurs substituants choisis dans l'ensemble constitué par un groupe hydroxy, un groupe amino primaire, et un groupe amino secondaire, est un composé amine représenté par la formule (11) suivante :

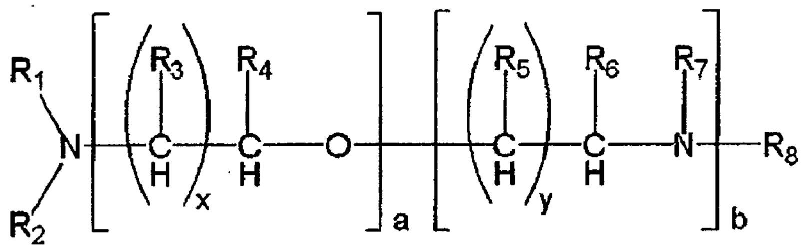

(11)

où, dans la formule (11) ci-dessus, chacun de $R_1$ à $R_8$, qui sont indépendants les uns des autres, est un atome d'hydrogène, un groupe hydroxyle, un groupe alkyle en $C_1$ à $C_{16}$, un groupe aryle en $C_6$ à $C_{16}$, un groupe hydroxyalkyle en $C_1$ à $C_{10}$, un groupe aminoalkyle en $C_1$ à $C_{10}$, un groupe monométhylaminoalkyle en $C_1$ à $C_{10}$ ou un groupe diméthylaminoalkyle en $C_1$ à $C_{10}$, x est un entier de 0 à 11, y est un entier de 0 à 11, a est un entier de 0 à 10 et b est un entier de 0 à 10, et
dans laquelle le composé amine représenté par la formule (11) ci-dessus est une ou plusieurs amines choisies dans l'ensemble constitué par la N,N-diméthyléthylènediamine, la N,N'-diméthyléthylènediamine, la N,N-diméthylpropylènediamine, la N,N'-diméthylpropylènediamine, la N,N-diméthylhexaméthylènediamine, la N,N'-diméthylhexaméthylènediamine, la triméthyldiéthylènetriamine, la triméthyléthylènediamine, la triméthylpropylènediamine, la triméthylhexaméthylènediamine, la tétraméthyldiéthylènetriamine, le N,N-diméthylaminoéthanol, le N,N-diméthylaminoisopropanol, la bis(3-diméthylaminopropyl)amine, la N-méthylpipérazine, le N,N-diméthylaminoéthoxyéthanol, la N,N,N'-triméthylaminoéthyléthanolamine, le N,N-diméthylaminoéthyl-N'-méthylaminoéthyl-N"-méthylaminoisopropanol, le N,N-diméthylaminoéthoxyéthoxyéthanol, la N,N-diméthyl-N',N'-bis(2-hydroxypropyl)-1,3-propanediamine, le N,N,N'-triméthyl-N'-(2-hydroxyéthyl)bis(2-aminoéthyl)éther, la N,N-bis(3-diméthylaminopropyl)-N-isopropanolamine, le N,N-diméthylaminohexanol et la N,N,N'-triméthyl-N'-(2-hydroxyéthyl)propylènediamine.

2. Composition de catalyseur pour la production d'une résine de polyuréthane selon la revendication 1, dans laquelle l'hydroxyalkyltriéthylènediamine ou hydroxytriéthylènediamine (A) est un composé amine représenté par la formule (2e) suivante :

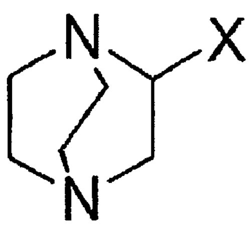

(2e)

où, dans la formule (2e) ci-dessus, X est un groupe hydroxyle, un groupe hydroxyméthyle ou un groupe hydroxyéthyle.

3. Composition de catalyseur pour la production d'une résine de polyuréthane selon la revendication 1 ou 2, dans laquelle le rapport de mélange de l'hydroxyalkyltriéthylènediamine ou hydroxytriéthylènediamine (A) au composé amine (B) ayant, dans sa molécule, un ou plusieurs substituants choisis dans l'ensemble constitué par un groupe hydroxy, un groupe amino primaire, et un groupe amino secondaire, est composé amine (A) / composé amine (B) = 1/99 à 99/1 en rapport en poids.

4. Procédé pour produire une résine de polyuréthane, qui comprend la réaction d'un polyol avec un polyisocyanate en présence de la composition de catalyseur telle que définie dans l'une quelconque des revendications 1 à 3.

5. Procédé pour produire une résine de polyuréthane selon la revendication 4, dans lequel la composition de catalyseur telle que définie dans l'une quelconque des revendications 1 à 3 est utilisée en une quantité située dans la plage allant de 0,01 à 30 parties en poids pour 100 parties en poids du polyol.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001504855 A
- AT 227268
- JP 46004846 A
- JP 61031727 B
- JP 2971979 B
- JP 63265909 A
- JP 2008045113 A
- JP 2003082051 A
- JP 2003105051 A
- JP 2003082052 A
- JP 2008142586 A
- JP 2008178990 A
- JP 2008185165 A
- JP 2008204535 A
- JP 2008278254 A
- JP 2008281558 A
- JP 2008296910 A
- JP 2008297912 A

### Non-patent literature cited in the description

- *Journal of Medicinal Chemistry*, 1993, vol. 36 (15), 2075-2083
- **KEIJI IWATA**. Polyurethane Resin Handbook. Nikkan Kogyo Shimbun, Ltd., 1987, 118
- *J. Med. Chem.*, 1993, vol. 36, 2075
- **GUNTER OERTEL**. Polyurethane Handbook. Hanser Publishers, 1985, 42-53
- **KEIJI IWATA**. Polyurethane Resin Handbook. Nikkan Kogyo Shimbun, Ltd., 1987, 117
- **GUNTER OERTEL**. Polyurethane Handbook. Hanser Publishers, 1985, 161-233
- **KEIJI IWATA**. Polyurethane Resin Handbook. Nikkan Kogyo Shimbun, Ltd., 1987, 150-221
- **GUNTER OERTEL**. Polyurethane Handbook. Hanser Publishers, 1985, 223-233
- **KEIJI IWATA**. Polyurethane Resin Handbook. Nikkan Kogyo Shimbun, Ltd., 1987, 211-221
- **GUNTER OERTEL**. Polyurethane Handbook. Hanser Publishers, 1985, 234-313
- **KEIJI IWATA**. Polyurethane Resin Handbook. Nikkan Kogyo Shimbun, Ltd., 1987, 224-283